# EUROPÄISCHE PATENTANMELDUNG

(11) **EP 2 090 301 A1**
(43) Veröffentlichungstag der Anmeldung: **19.08.2009**
(21) Anmeldenummer: 09152690.5
(22) Anmeldetag: 12.02.2009
(51) Int. Cl.: A61K 31/12, A61P 17/04, A61P 17/08, A61P 17/00, A61K 8/35

(54) **3-(4-Hydroxy-3-methoxyphenyl)-1-(4-hydroxyphenyl)-1-propanon und seine Verwendung in kosmetischen und pharmazeutischen Zubereitungen**

(30) Priorität: 18.02.2008 DE 102008009929
(71) Anmelder: Symrise GmbH & Co. KG, 37603 Holzminden (DE)
(72) Erfinder: Schmaus, Dr. Gerhard, 37671 Höxter-Bosseborn (DE); Koch, Dr. Oskar, 37079 Göttingen (DE)
(74) Vertreter: Sendrowski, Heiko

(57) **Zusammenfassung**

Die vorliegende Erfindung betrifft Verwendungen der Verbindung der Formel 1 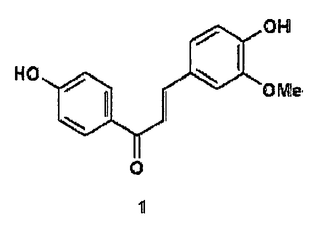 in kosmetischen oder pharmazeutischen und medizinischen Zubereitungen.

## Beschreibung

Die vorliegende Erfindung betrifft Verwendungen der Verbindung der Formel 1 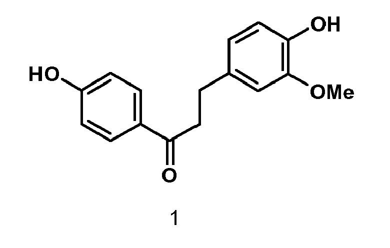 in kosmetischen oder pharmazeutischen Zubereitungen.

Die Haut, insbesondere die Epidermis, ist als Barriereorgan des menschlichen Organismus in besonderem Maße äußeren Einwirkungen unterworfen. Viele intrinsische Faktoren (z.B. genetische Veranlagung und/oder mit dem natürlichen Alterungsprozeß verbundene Veränderungen) und extrinsische Faktoren (z.B. Schädigung der Hautbarriere, Einwirkung von UV-Licht, irritierenden oder allergie-auslösenden Substanzen, mechanische Einwirkung z.B. durch Rasur) können zu einer Hautirritation führen.

Unter Hautirritation ist im Zusammenhang mit dieser Anmeldung jede Veränderung der Haut zu verstehen, die bei Mensch oder Tier sensorisches Missempfinden auslöst oder/und durch ein trockenes, gerötetes und/oder entzündetes Hautbild geprägt ist. Dabei umfasst der Begriff sensorisches Missempfinden selbstverständlich auch Zustände wie Jucken oder Schmerz. Hautirritation kann insbesondere phänomenologisch verschiedene Hautzustände umfassen: Sensible Haut, empfindliche Haut inklusive empfindlicher Kopfhaut, verletzliche Haut, atopische Haut, irritierte Haut, Rosacea, entzündete Haut, die sich im jeweils höheren Schweregrad in einer Hautrötung, dem sogenannten Erythem äußert.

Das Problem "sensible Haut" betrifft eine wachsende Anzahl Erwachsener und Kinder. Man geht inzwischen davon aus, dass bis zu 50 % der Bevölkerung eine sensible Haut haben (L. Misery et al., Ann. Dermatol. Venereol. 2005, 132, 425-429). Mit sensibler Haut bezeichnet man eine Haut mit einer erniedrigten Reizschwelle für Irritantien, wie hyperreaktive und intolerante, aber auch atopische Haut. Bei Menschen mit sensibler, empfindlicher oder verletzlicher Haut kann ein mit "Stinging" (engl. "to sting" = verletzen, brennen, schmerzen) bezeichnetes Phänomen beobachtet werden. Typische, mit den Begriffen "Stinging" oder "empfindlicher Haut" in Verbindung gebrachte, störende Phänomene sind Hautrötung, Kribbeln, Prickeln, Spannen und Brennen der Haut und Juckreiz. Sie können durch stimulierende Umgebungsbedingungen wie z. B. Massage, Tensideinwirkung, Einwirkung weiterer chemischer Substanzen wie z. B. Milchsäure, Wettereinfluss wie Wärme, Kälte, Trockenheit, aber auch feuchte Wärme, Wärmestrahlung und UV-Strahlung, z. B. der Sonne, oder psychologischen Stress hervorgerufen werden.

"Empfindliche" Kopfhaut ist ebenfalls gekennzeichnet durch Hautrötung, Kribbeln, Prickeln, Brennen und Juckreiz. Auslöser sind beispielsweise Seife, Shampoos oder andere Haarpflegemittel, Tenside, hartes Wasser mit hohen Kalkkonzentrationen und/oder mechanische Beanspruchung. Erytheme und Hyperseborrhoe (übermäßige Talgproduktion) der Kopfhaut sowie Schuppen sind häufig mit den beschriebenen Phänomenen assoziiert.

Bei ca. 10-20% der Bevölkerung industrieller Länder, mit steigender Tendenz, ist die Atopie zu beobachten, eine familiär auftretende Überempfindlichkeit der Haut und der Schleimhäute gegen Umweltstoffe, mit gesteigerter Bereitschaft, gegen Substanzen aus der natürlichen Umwelt Überempfindlichkeitsreaktionen vom Soforttyp (Allergien) zu entwickeln. Atopie ist vermutlich genetisch bedingt. Atopie kann sich als atopische Dermatitis äußern. Dabei ist die Hautbarriere geschädigt, die Haut ist oft entzündet und juckt.

Die erythematöse Wirkung des ultravioletten Teils der Sonnenstrahlung oder künstlicher Strahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen. Erythematöse Hauterscheinungen treten auch als Begleiterscheinungen bei gewissen Hauterkrankungen oder -unregelmässigkeiten auf, beispielsweise Akne, bakteriell induzierte Entzündungen der Haut, Kutanreaktionen, Dermographismus, das Wundsein der Haut im allgemeinen, Hautmaulwurf, Wundrose, Gürtelrose, Erfrierungen oder Verbrennungen. Eine besondere Form der Hautrötung stellt dabei die Rosacea dar. Antiinflammatorisch wirksame Zubereitungen können daher auch vorteilhaft eingesetzt werden allgemein krankheitsbedingte Hautrötungen und im speziellen die Rosacea abklingen zu lassen, über einen längeren Zeitraum zu unterdrücken und das wieder Auftreten weitgehend zu verhindern.

Hautentzündungen, die mit Rötung und Juckreiz einhergehen, werden ebenfalls durch Insektenstiche hervorgerufen. Antinflammatorisch wirkende Mittel können daher auch dazu beitragen die Folgen eines Insektenstiches, wie z. B. Rötung, Quaddelbildung, Juckreiz und schmerzhafte Schwellungen der Haut, abklingen zu lassen, über einen längeren Zeitraum zu unterdrücken und das wieder Auftreten zu verhindern.

Erytheme treten auch verstärkt im Windelbereich von Kleinkindern, um so mehr von Säuglingen auf (Windel Dematitis). Auch Inkontinenz, ein besonders im höheren Alter verstärkt auftretendes Leiden, ist häufig mit Erythemen und Hautrötung als Folge dauernder Exposition mit Feuchtigkeit und Reizstoffen verbunden (Inkontinenz Dermatitis).

Zwar werden in den angesprochenen technischen Bereichen bereits eine Vielzahl Wirkstoffe mit hautirritationsverringernder Wirkung eingesetzt, doch wird weiterhin nach Alternativen gesucht.

In der kosmetischen und pharmazeutischen Industrie besteht daher ein beständiger Bedarf an Mitteln mit hautirritationsverringernder Wirkung. Bei der Suche nach entsprechenden antiinflammatorisch wirksamen Mitteln ist dabei zu beachten, dass die in kosmetischen und/oder pharmazeutischen Produkten verwendeten Substanzen im weiteren
- toxikologisch unbedenklich,
- gut hautverträglich,
- stabil (insbesondere in üblichen kosmetischen und/oder pharmazeutischen Formulierungen),
- vorzugsweise schwach riechend oder (weitgehend) geruchlos,
- vorzugsweise farblos und nicht verfärbend, und
- preiswert herstellbar (d.h. unter Einsatz von Standardverfahren und/oder ausgehend von Standardprekursoren)
sein müssen.

Die Suche nach geeigneten (Wirk-)substanzen, die eine oder mehrere der genannten Eigenschaften in ausreichendem Maße besitzen, ist dem Fachmann dadurch erschwert, dass keine klare Abhängigkeit zwischen der chemischen Struktur einer Substanz einerseits und ihrer antiinflammatorischen Aktivität sowie ihrer Stabilität andererseits besteht. Des Weiteren gibt es keinen vorhersehbaren Zusammenhang zwischen der antiinflammatorischen Wirkung, der toxikologischen Unbedenklichkeit, der Hautverträglichkeit und/oder der Stabilität.

Es war daher die Aufgabe der vorliegenden Erfindung, einen antiinflammatorischen Wirkstoff anzugeben, der vorzugsweise eine oder mehrere der vorab genannten Nebenbedingungen und besonders bevorzugt alle der vorab genannten Bedingungen erfüllt.

Die Aufgabe der vorliegenden Erfindung wird gelöst durch die Gegenstände der unabhängigen Ansprüche. Die bevorzugten Ausgestaltungen werden in den abhängigen Ansprüchen sowie in der nachfolgenden Beschreibung dargestellt. Hierbei sind die bevorzugten erfindungsgemäßen Ausgestaltungen untereinander auch kombinierbar.

Erfindungsgemäß einzusetzen ist die Verbindung der Formel 1

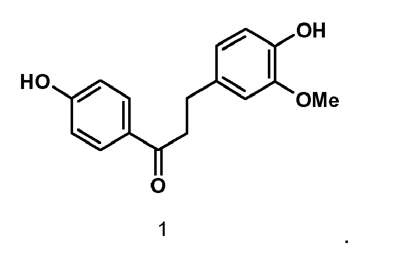

Im Sinne der vorliegenden Erfindung und deren bevorzugten Ausgestaltungen werden unter den Begriffen "Verbindung der Formel 1" und "3-(4-Hydroxy-3-methoxyphenyl)-1-(4-hydroxyphenyl)-1-propanon" auch deren Solvate, bevorzugt Hydrate, und Salze, bevorzugt Phenolatsalze, verstanden.

Ein Gegenstand der vorliegenden Erfindung betrifft somit eine kosmetische oder pharmazeutische Zubereitung umfassend oder bestehend aus den folgenden Komponenten:
a) eine antiinflammatorisch wirksame Menge einer Verbindung der Formel 1 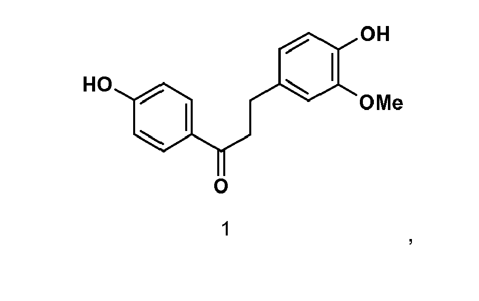
b) gegebenenfalls ein, zwei, drei, vier oder mehrere weitere antiinflammatorisch wirksame Verbindungen,
   sowie
c) eine oder mehrere zu Komponenten (a) und (b) kompatible kosmetische und/oder pharmazeutische Grund-, Hilfs- oder Zusatzstoffe.

Ein weiterer Gegenstand der vorliegenden Erfindung betrifft eine Verbindung der Formel 1 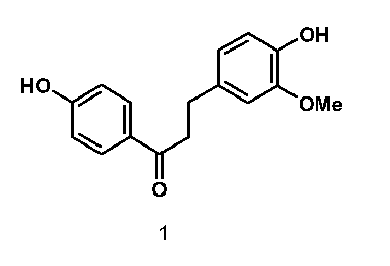 zur Verwendung als Arzneimittel.

Ein weiterer Gegenstand der vorliegenden Erfindung betrifft eine Verwendung einer Verbindung der Formel 1 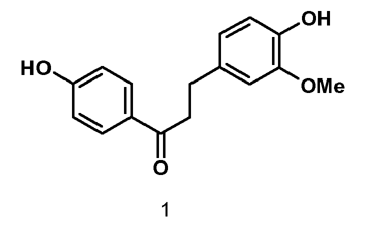 in einer antiinflammatorisch wirksamen Menge in kosmetischen Zubereitungen.

Ein weiterer Gegenstand der vorliegenden Erfindung betrifft eine Verwendung einer Verbindung der Formel 1 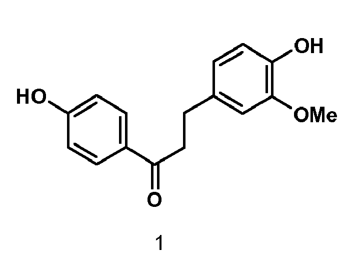 in einer antiinflammatorisch wirksamen Menge zur Herstellung eines Arzneimittels zur Behandlung oder Vorbeugung von Inflammationen.

Ein weiterer Gegenstand der vorliegenden Erfindung betrifft eine Verwendung einer Verbindung der Formel 1 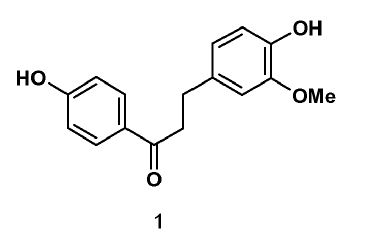 in einer antiinflammatorisch wirksamen Menge zur Behandlung oder Vorbeugung von Inflammationen.

Ein weiterer Gegenstand der vorliegenden Erfindung betrifft eine Verwendung einer antiinflammatorisch wirksamen Menge einer Verbindung der Formel 1 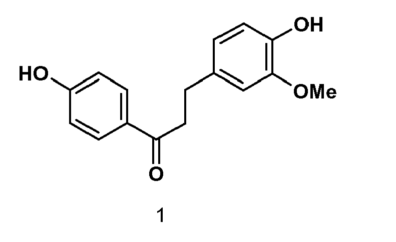 oder einer erfindungsgemäßen kosmetischen oder pharmazeutischen Zubereitung zum Verringern, Aufheben oder Unterdrücken einer inflammatorischen Wirkung einer, zwei, drei, vier oder mehreren Verbindungen auf die menschliche oder tierische Haut.

Ein weiterer Gegenstand der vorliegenden Erfindung betrifft ein Verfahren zur kosmetischen Behandlung oder Vorbeugung inflammatorischer Prozesse der Haut umfassend oder bestehend aus der topischen Applikation einer antiinflammatorisch wirksamen Menge der erfindungsgemäßen kosmetischen oder pharmazeutischen Zubereitung auf die Haut eines menschlichen oder tierischen Körpers.

Ein weiterer Gegenstand der vorliegenden Erfindung betrifft ein Verfahren zum Behandeln oder Vorbeugen einer inflammatorischen Wirkung einer, zwei, drei, vier oder mehreren Verbindungen auf die menschliche oder tierische Haut umfassend oder bestehend aus folgenden Schritten:
a) Bereitstellen einer, zwei, drei, vier oder mehreren Verbindungen, die eine inflammatorische Wirkung auf die Haut ausüben,
b) Bereitstellen einer Verbindung der Formel 1 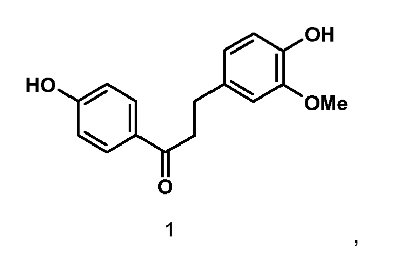
c) gegebenenfalls Bereitstellen eines, zwei, drei, vier oder mehrerer Grund-, Hilfs- und/oder Zusatzstoffe für kosmetische oder pharmazeutische Zubereitungen und
d) Vermischen der Verbindungen aus Schritt a) mit der Verbindung der Formel 1 aus Schritt b) und gegebenenfalls der Grund-, Hilfs- und/oder Zusatzstoffe aus Schritt c) zu einer Zubereitung,
   wobei ein oder mehrere Verbindungen aus Schritt a) in einer Menge bezogen auf die fertige Zubereitung umfasst sind, die in einer Zubereitung ohne die Verbindung der Formel 1 eine inflammatorische Wirkung auf die menschliche oder tierische Haut vermitteln und
   wobei die Verbindung der Formel 1 in einer Menge umfasst ist, die ausreicht die inflammatorische Wirkung der Verbindungen aus Schritt a) auf die Haut zu verringern, aufzuheben oder zu unterdrücken.

Ein weiterer Gegenstand der vorliegenden Erfindung betrifft eine Duftstoffkomposition, umfassend oder bestehend aus
a) einer sensorisch wirksamen Menge eines Duftstoffes oder Duftstoffgemisches,
b) einer antiinflammatorisch wirksamen Menge einer Verbindung der Formel 1 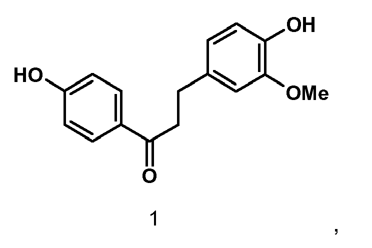
c) gegebenenfalls ein, zwei, drei, vier oder mehreren weiteren antiinflammatorisch wirksamen Verbindungen und
d) gegebenenfalls ein oder mehreren Grund-, Hilfs- und/oder Zusatzstoffen.

Die vorliegende Erfindung beruht auf der überraschenden Erkenntnis, dass die Verbindung der Formel 1 hervorragende antiinflammatorische Eigenschaften besitzt und daher hervorragend insbesondere in einer antiinflammatorisch wirksamen Menge in kosmetischen oder pharmazeutischen Zubereitungen verwendet werden kann. Somit kann die Verbindung der Formel 1 verwendet werden, um Inflammationen zu behandeln oder diesen vorzubeugen. Solche Inflammationen werden bevorzugt ausgelöst durch extrinsische Faktoren, bevorzugt durch mechanische Reizung, chemische Reizung sowie Reizung durch Sonneneinstrahlung; und/oder intrinsische Faktoren, bevorzugt genetische Faktoren und natürliche Alterungsprozesse.

Die vorliegende Erfindung beruht des weiteren auf der überraschenden Erkenntnis, dass durch die Verwendung der Verbindung der Formel 1 in Kombination mit ein, zwei, drei, vier oder mehreren Verbindungen, welche eine inflammatorische, bevorzugt hautirritierende und/oder hautrötende Wirkung auf die menschliche oder tierische Haut haben, die inflammatorische Wirkung dieser Verbindungen zumindest teilweise verringert, aufgehoben oder unterdrückt werden kann. Folglich können erfindungsgemäß auch Verbindungen mit einer inflammatorischen Wirkung auf die menschliche oder tierische Haut in kosmetischen oder pharmazeutischen Zubereitung verwendet werden.

Die Untersuchungen zu den chemischen Eigenschaften zeigten, dass sich die Verbindung der Formel 1 durch hohe Stabilität, insbesondere durch hohe Temperaturstabilität, durch hohe Stabilität in einem breiten pH-Wert Bereich sowie durch hohe Photostabilität auszeichnet, wodurch sie in hervorragender Weise in unterschiedlichsten kosmetische Zubereitungen und pharmazeutischen Produkten (Arzneimittel, Medizinprodukte) einsetzbar ist. Die erfindungsgemäße Verbindung der Formel 1 stellt in reiner Form einen weißen Feststoff dar, der sich farblos in kosmetischen und/oder pharmazeutischen Grund-, Hilfs- oder Zusatzstoffe, bevorzugt Trägerstoffen löst. Ferner wurden nach Einarbeitung der erfindungsgemäßen Verbindung der Formel 1 in erfindungsgemäße kosmetischen und pharmazeutischen Zubereitungen keine Verfärbungen dieser Zubereitung festgestellt.

In DE 10 2007 035 139.0 (Symrise GmbH & Co. KG) wird 3-(4-Hydroxy-3-methoxyphenyl)-1-(4-hydroxyphenyl)-1-propanon, entsprechend der Verbindung der Formel 1 der vorliegenden Erfindung, ein Verfahren zu dessen Herstellung sowie die Verwendung von 3-(4-Hydroxy-3-methoxyphenyl)-1-(4-hydroxyphenyl)-1-propanon als antimikrobieller Wirkstoff beschrieben. Es wird in DE 10 2007 035 139.0 nicht offenbart, dass 3-(4-Hydroxy-3-methoxyphenyl)-1-(4-hydroxyphenyl)-1-propanon, entsprechend der Verbindung der Formel 1 der vorliegenden Erfindung, eine antiinflammatorische Wirksamkeit aufweist.

In US Provisional Application 60/951,741 (Symrise GmbH & Co. KG) wird 3-(4-Hydroxy-3-methoxyphenyl)-1-(4-hydroxyphenyl)-1-propanon, entsprechend der Verbindung der Formel 1 der vorliegenden Erfindung, ein Verfahren zu dessen Herstellung sowie die Verwendung von 3-(4-Hydroxy-3-methoxyphenyl)-1-(4-hydroxyphenyl)-1-propanon als Antioxidans beschrieben. Es wird in US 60/951,741 nicht offenbart, dass 3-(4-Hydroxy-3-methoxyphenyl)-1-(4-hydroxyphenyl)-1-propanon, entsprechend der Verbindung der Formel 1 der vorliegenden Erfindung, eine antiinflammatorische Wirksamkeit aufweist.

Die starke antiinflammatorische Wirksamkeit der erfindungsgemäßen Verbindung der Formel 1 beruht unter anderem auf deren Eigenschaft die durch extrinsische und intrinsische Faktoren hervorgerufene Hochregulation von Entzündungsmediatoren, unter anderem Interleukine (insbesondere IL-1 und IL-6), PGE-2 (ProstaglandinE2) und insbesondere TNF-alpha (Tumor Nekrose Faktor alpha), dosisabhängig bereits in geringer Konzentration zu hemmen. Sie kann somit hervorragend als Alternative oder in Ergänzung zu bereits bekannten weiteren antiinflammatorischen Verbindungen (Wirkstoffen) in kosmetischen und pharmazeutischen Zubereitungen oder dergleichen als antiinflammatorischer Wirkstoff Einsatz finden.

Über diese hier erstmals beschriebenen und damit erfindungsgemäßen antiinflammatorischen Eigenschaften von 3-(4-Hydroxy-3-methoxyphenyl)-1-(4-hydroxyphenyl)-1-propanon sowie über dessen Verwendung in erfindungsgemäßen kosmetischen und pharmazeutischen, bevorzugt dermatologischen Zubereitungen zur Vorbeugung gegen Hautirritation und/oder zur Behandlung von Inflammationen, bevorzugt Hautirritationen und Hautrötungen (Erytheme) sowie weiterer unerwünschter, mit inflammatorischen Prozessen einhergehenden Veränderungen, war bis dato nichts bekannt.

Unter dem Begriff "Inflammationen" (Entzündungen) werden in der vorliegenden Erfindung Hautirritationen und/oder Hautrötungen (Erytheme) sowie weitere (sekundäre) unerwünschte, mit inflammatorischen Prozessen einhergehenden Veränderungen verstanden.

Unter dem Begriff "Haut" ist gemäß vorliegenden Erfindung auch Schleimhaut umfasst.

Unter dem Begriff "Hautirritation" ist im Zusammenhang mit dieser Erfindung jede Veränderung der Haut zu verstehen, die bei Mensch oder Tier sensorisches Missempfinden auslöst oder/und durch ein trockenes, gerötetes und/oder entzündetes Hautbild geprägt ist. Dabei umfasst der Begriff "sensorisches Missempfinden" selbstverständlich auch Zustände wie Jucken oder Schmerz. Erfindungsgemäße Hautirritation kann insbesondere phänomenologisch verschiedene Hautzustände umfassen: Sensible Haut, empfindliche Haut inklusive empfindlicher Kopfhaut, verletzliche Haut, atopische Haut, irritierte Haut, Rosacea, entzündete Haut, die sich im jeweils höheren Schweregrad in einer Hautrötung, dem sogenannten Erythem äußert.

Unter dem Begriff "Hautrötung" bzw. "Erythem" ist im Zusammenhang mit der vorliegenden Erfindung üblicherweise eine Hautrötung gemeint, die durch ultraviolette Strahlung (natürlich durch Sonnenstrahlung oder durch künstliche Strahlung) auf die Haut entsteht, aber auch als Begleiterscheinungen bei gewissen Hauterkrankungen oder -unregelmässigkeiten auftritt.

Beispielsweise stellt die erfindungsgemäße "Hautrötung" auch der typische Hautausschlag beim Erscheinungsbild der Akne dar, der infolge der damit einhergehenden entzündlichen Prozesse entsteht und das Wohlbefinden der Betroffenen selbst in leichten Fällen beeinträchtigt. Zu weiteren Hautanomalien, die mit vorübergehender oder dauerhafter erfindungsgemäßer Rötung der Haut einhergehen, zählen bevorzugt die bakteriell induzierte Entzündung der Haut, Kutanreaktionen, Dermographismus, das Wundsein der Haut im allgemeinen, Hautmaulwurf, Wundrose, Gürtelrose, Erfrierungen oder Verbrennungen. Eine besondere Form der Hautrötung stellt dabei die Rosacea dar. Rosacea [zu lateinisch rosaceus >rosenfarbig<], auch Kupferfinnen oder Rotfinnen genannt, ist eine meist erst im mittleren und höheren Lebensalter auftretende chronische Erkrankung der Gesichtshaut, die besonders den Bereich der Nase, auch der Stirn und der Wangen (Schmetterlingsfigur) betrifft. Durch Erweiterung der oberflächlichen Hautgefässe kommt es zu teils tiefroten Blutstauungen, zu Gefässerweiterungen, je nach Form auch zu Hautschuppung, schubweiser Ausbildung von Pappeln oder Pusteln und (meist nur bei Männern) später zur Ausbildung eines Rhinophyms. Hautrötungen im allgemeinen und Rosacea im besonderen, stellen eine hohe physische und psychische Belastung für den Betroffenen dar. Auch werden sie vom Betrachter meist als optisch wenig attraktiv, krankhaft und unästhetisch wahrgenommen.

Erfindungsgemäße Inflammationen der Haut (Hautentzündungen), die mit Rötung und Juckreiz einhergehen, können ebenfalls durch Insektenstiche hervorgerufen werden. Die Reaktion auf Insektenstiche ist in der Regel eine harmlose örtliche Hautveränderung als Folge eines Stiches vor allem von Stechmücken, Stechfliegen, Läusen, Flöhen und Wanzen. Die Hautveränderung selbst besteht meist in juckenden, auch schmerzenden Hautschwellungen. Die Stiche von Bienen, Wespen, Hornissen und Hummeln, die durch Toxine und Allergene wirken, führen je nach Empfindlichkeit des Organismus und der Lage der Einstichstelle zu Rötung, Quaddelbildung und schmerzhaften Schwellungen an der Einstichstelle.

Erfindungsgemäße Hautrötungen (Erytheme) treten auch verstärkt im Windelbereich von Kleinkindern, um so mehr von Säuglingen auf (Windel Dematitis). Auch Inkontinenz, ein besonders im Alter verstärkt auftretendes Leiden, ist häufig mit Erythemen und Hautrötung als Folge dauernder Exposition mit Feuchtigkeit und Reizstoffen verbunden (Inkontinenz Dermatitis).

Unter dem Begriff "hautirritationsverringernder Wirkung" ist im Zusammenhang mit dieser Erfindung das Abmildern, Verrringern, Abstellen oder Verhindern von Hautirritationen und/oder Hautrötungen, insbesondere wie oben beschrieben, zu verstehen. Dabei basiert die hautirritations- und/oder hautrötungsverringernde Wirkung insbesondere auf Hautberuhigung, Entzündungshemmung und/oder Rötungslinderung.

Erfindungsgemäße kosmetische oder pharmazeutische Zubereitungen sind bevorzugt, **dadurch gekennzeichnet, dass** die Zubereitung zusätzlich
d) eine wirksame Menge eines, zwei, drei, vier oder mehrerer UV-Filter umfasst, so dass der Lichtschutzfaktor der kosmetischen oder pharmazeutischen Zubereitung >2 beträgt.

Eine weitere bevorzugte Ausführungsform erfindungsgemäßer kosmetischer oder pharmazeutischer Zubereitung ist **dadurch gekennzeichnet, dass** die Zubereitung zusätzlich
e) ein, zwei, drei, vier oder mehrere Verbindungen in einer Menge bezogen auf die fertige Zubereitung umfasst, die in einer Zubereitung ohne die Verbindung der Formel 1 eine inflammatorische Wirkung auf die menschliche oder tierische Haut vermittelt,
wobei die Menge der Verbindung der Formel 1 bezogen auf die fertige Zubereitung ausreicht, die inflammatorische Wirkung der Verbindungen aus e) zu verringern, aufzuheben oder zu unterdrücken.

Im Sinne der vorliegenden Erfindung sind die ein, zwei, drei, vier oder mehreren Verbindungen aus Merkmal e), die eine inflammatorische Wirkung auf die menschliche oder tierische Haut vermitteln (potentiell irritierende Verbindungen), üblicherweise anorganische oder organische Verbindungen synthetischen oder natürlichen Ursprungs, die in kosmetischen oder pharmazeutischen Zubereitungen verwendet werden können und individuell je nach Einsatzkonzentration und Hautzustand ein Hautirritationspotential besitzen. Solche hautirritierende Verbindungen werden bevorzugt gemäß deutschem Chemikaliengesetz mit den R-Sätzen R36, R37 und/oder R38 versehen.

Verbindungen, die erfindungsgemäß eine Inflammation der Haut hervorrufen können, sind üblicherweise in den verschiedensten Verbindungsgruppen zu finden, beispielsweise Detergentien, Mittel zur Verbesserung der Hautgeschmeidigkeit, Mittel zur Behandlung von Akne, Hautaufheller, Konservierungsmittel, Trägerstoffe und Duftstoffe.

Verbindungen, die erfindungsgemäß eine Inflammation der Haut hervorrufen können, werden bevorzugt ausgewählt aus der Gruppe bestehend aus
- Detergentien, bevorzugt Natriumlaurylsulfat oder Seifen;
- Mittel zur Verbesserung der Hautgeschmeidigkeit, bevorzugt aliphatische und aromatische alpha-Hydroxysäuren, beta-Hydroxysäuren oder Ketosäuren, bevorzugt Glycolsäure, Milchsäure und Salicylsäure, sowie deren Derivate, die bevorzugte ausgewählt werden aus der Gruppe bestehend aus Amiden, Lactonen, Anhydriden und Ester, sowie ebenfalls deren organische und anorganische Salze;
- Mittel zur Behandlung von Akne, bevorzugt Peroxide, weiter bevorzugt Benzoylperoxid; Salicylsäure; Resorcinol; und Retinol-Derivate, bevorzugt Retinsäure, Retinaldehyd, Retinol, Retinylacetat und Retinylpalmitat;
- Hautaufheller, bevorzugt Hydrochinon; Kojisäure; Resorcinol und Derivate hiervon;
- Konservierungsmittel, bevorzugt Parabene, bevorzugt Isobutylparaben und Propylparaben; Phenoxyethanol; und
- Harnstoff.

Die vorstehend genannten Verbindungen, die erfindungsgemäß eine Inflammation der Haut hervorrufen, werden in erfindungsgemäßen kosmetischen oder pharmazeutischen Zubereitungen bevorzugt in einer Konzentration eingesetzt, in der sie ohne den Einsatz der Verbindung der Formel 1 eine Inflammation der menschlichen oder tierischen Haut hervorrufen.

In der nachfolgenden Tabelle 1 werden übliche Dosisangaben für ausgewählte potentiell irritierende Verbindungen in kosmetischen Formulierungen dargestellt.

| **Potentiell irritierende Verbindung** | **Dosisangabe bezogen auf fertige kosmetische Verbindung [Gew.-%]** |
|---|---|
| Glycolsäure | 0,5 - 5,0 |
| Milchsäure | 0,5 - 5,0 |
| Salicylsäure | 0,5 - 5,0 |
| Retinsäure | 0,1 - 1 |
| Retinol | 0,1 - 1 |
| Retinylpalmitat | 0,1 - 1 |
| Hydrochinon | 0,1 - 1 |
| Kojisäure | 0,1 - 1 |
| Resorcinol | 0,1 - 1 |
| Benzoylperoxid | 0,1 - 5 |
| Phenoxyethanol | 0,1 - 5 |
| Propylparaben | 0,1 - 0,5 |
| Isobutylparaben | 0,1 - 0,5 |

Eine weitere bevorzugte Ausgestaltung der erfindungsgemäßen kosmetischen oder pharmazeutischen Zubereitung ist **dadurch gekennzeichnet, dass** die Zubereitung ausgewählt wird aus der Gruppe bestehend aus flüssigen Formulierungen, bevorzugt Reinigungslösungen oder Tränkflüssigkeiten; halbfesten Formulierungen, bevorzugt Salben und Cremes.

Da es sich im Sinne der vorliegenden Erfindung um die Applikation von der Verbindung der Formel 1 bzw. kosmetischer Zubereitungen, pharmazeutischen, bevorzugt dermatologischer Zubereitungen umfassend die Verbindung der Formel 1 zur Behandlung oder Vorbeugung von Inflammationen der Haut handelt, wird die Applikation jeweils bevorzugt topisch auf die Haut, insbesondere Schleimhaut des menschlichen oder tierischen Körpers vorgenommen. Hierbei werden erfindungsgemäß die durch extrinsische oder intrinsische Faktoren hervorgerufene Inflammationen der Haut, bevorzugt Hautirritationen und Hautrötungen (Erythembildung), die u.a. mit Juckreiz und Quaddelbildung verbunden sein können, entweder gehemmt und/oder komplett unterbunden.

Die erfindungsgemäße pharmazeutische Zubereitung (Arzneimittel oder Medizinprodukt) lässt sich auf dem Gebiet der Human- und Veterinärmedizin gegen eine Vielzahl von Krankheiten einsetzen, wie zum Beispiel Urticaria, Kontaktdermatitis, Rosacea, Atopie sowie allgemein gegen alle Entzündungsprozesse, auch Zahn(fleisch)entzündungen wie Parodonthose.

Die Einsatzkonzentration der erfindungsgemäßen Verbindung der Formel 1 in einer erfindungsgemäßen kosmetischen und pharmazeutischen Zubereitung oder einer erfindungsgemäßen pharmazeutischen Zubereitung liegt dabei üblicherweise im Bereich von 0,001 und 20 Gew.-%, vorzugsweise im Bereich zwischen 0,001 bis 10 Gew.-%, bevorzugt im Bereich zwischen 0,005 und 5 Gew.-% und besonders bevorzugt im Bereich zwischen 0,01 und 1,0 Gew.-%, jeweils bezogen auf die Gesamtmasse der kosmetischen oder pharmazeutischen Zubereitung.

Die Verbindung der Formel 1 war selbst bei einer Testkonzentration von 50 Gew.-% nicht sensibilisierend und nicht irritierend.

Die erfindungsgemäß antiinflammatorisch wirksame Verbindung der Formel 1 kann auch als Bestandteil von erfindungsgemäßen Duftstoffkompositionen (Riechstoffkompositionen) eingesetzt werden und beispielsweise einem parfümierten Fertigprodukt eine antiinflammatorische Wirkung verleihen. Da der Anteil an Parfüm in einem kosmetischen Fertigprodukt häufig im Bereich von ca. 1 Gew.-% liegt, wird ein Parfüm, welches die erfindungsgemäße Verbindung der Formel 1 enthält, vorzugsweise zu etwa 1 - 50 Gew.-% aus der Verbindung der Formel 1 umfassen oder daraus bestehen.

Als besonders vorteilhaft hat es sich erwiesen, dass die Verbindung der Formel 1 einen schwachen, angenehm an Vanilleschote erinnernden Eigengeruch besitzt; denn diese Eigenschaft prädestiniert sie besonders für den Einsatz als antiinflammatorischer Wirkstoff in einer Duftstoffkomposition. Darüber hinaus weist die Verbindung aufgrund ihrer Struktur ein hohes Haftvermögen auf Haut und Haar auf wodurch insbesondere auch bei Anwendungen in "rinse off" Produkten zur Pflege und Behandlung der Kopfhaut und von Haar ein besonderes nachhaltiger und langanhaltender antiinflammatorischer Effekt erzielt werden kann.

Das erfindungsgemäß antiinflammatorisch wirksame 3-(4-Hydroxy-3-methoxyphenyl)-1-(4-hydroxyphenyl)-1-propanon kann mit einem oder mehreren pharmazeutisch und/oder kosmetisch akzeptablen Grund-, Hilfs- oder Zusatzstoffen weiterverarbeitet werden, bevorzugt zu einer erfindungsgemäßen Zubereitung in Feststoffform, wobei die Verbindung der Formel 1 bevorzugt mit einem festen Träger versetzt und anschließend durch geeignete Verfahren getrocknet wird. Pharmazeutisch bzw. kosmetisch kompatibel bzw. akzeptable sind solche Grund-, Hilfs- und Zusatzstoffe, insbesondere auch Trägerstoffe, die für den menschlichen oder tierischen Organismus, an dem sie angewendet werden sollen, zumindest keine toxischen Effekte aufweisen.

Das erfindungsgemäß antiinflammatorisch wirksame 3-(4-Hydroxy-3-methoxyphenyl)-1-(4-hydroxyphenyl)-1-propanon kann auch weiterverarbeitet werden zu einer erfindungsgemäßen verdünnten Zubereitung in Flüssigform, indem die Mischung optional mit einem pharmazeutisch und/oder kosmetisch akzeptablen Lösungsmittel wie z. B. Neutralöl, Mineralöl, Silikonöl, pflanzlichen Ölen, Fettalkoholen, Fettsäureestern, Ethanol, 1,2-Propylenglykol, 1,3-Butylenglykol, 1,2-Pentandiol und Wasser sowie Mischungen zweier oder mehrerer der genannten Lösungsmittel versetzt wird. Solche erfindungsgemäß hergestellten Zubereitungen sind insbesondere für kosmetische Zwecke gut weiterverarbeitbar. Gegebenenfalls können diese erfindungsgemäßen Zubereitungen hergestellt werden unter Zusatz eines Lösungsvermittlers, Konservierungsmittels oder Antioxidans.

Weiterhin kann das erfindungsgemäße, antiinflammatorisch wirksame 3-(4-Hydroxy-3-methoxyphenyl)-1-(4-hydroxyphenyl)-1-propanon oder die dieses enthaltende flüssige oder feste Zubereitung auch durch Verkapselung weiterverarbeitet werden. Erfindungsgemäß wird antiinflammatorisch wirksames 3-(4-Hydroxy-3-methoxyphenyl)-1-(4-hydroxyphenyl)-1-propanon sowie die dieses enthaltende flüssige oder feste Zubereitung verkapselt mit einem festen Hüllmaterial, das vorzugsweise ausgewählt ist aus Stärken, abgebaute oder chemisch oder physikalisch modifizierte Stärken (insbesondere Dextrine und Maltodextrine), Gelatinen, Wachsmaterialien, Liposomen, Gummi Arabicum, Agar-Agar, Ghatti-Gummi, Gellan Gum, modifizierte und nicht modifizierte Cellulosen, Pullulan, Curdlan, Carrageenane, Alginsäure, Alginate, Pektin, Inulin, Xanthan Gum und Mischungen zweier oder mehrerer der genannten Substanzen.

Wesentliche Anwendungsgebiete für antiinflammatorisch wirksames 3-(4-Hydroxy-3-methoxyphenyl)-1-(4-hydroxyphenyl)-1-propanon sind kosmetische (topische) Zubereitungen, die abgesehen von der Anwesenheit der Verbindung der Formel 1 wie üblich zusammengesetzt sind und dem kosmetischen Lichtschutz, zur Behandlung, der Pflege und der Reinigung der Haut und/oder der Haare oder als Schminkprodukt in der dekorativen Kosmetik dienen. Entsprechend können derartige Zubereitungen z. B. als Reinigungsmittel wie z. B. Seife, Syndet, flüssige Wasch-, Dusch-, und Badepräparat, Hautpflegemittel wie z. B. Emulsion (als Lösung, Dispersion, Suspension; Creme, Lotion oder Milch je nach Herstellungsverfahren und Inhaltsstoffen vom Typ W/O, O/W oder multiple Emulsion, PIT Emulsion, Emulsionsschaum, Mikro-, Nanoemulsion, Pickering Emulsion), Salbe, Paste, Gel (inklusive Hydro-, Hydrodispersions-, Oleogel), alkoholische oder wässrig/alkoholische Lösung, Öl, Toner, Balsam, Serum, Puder, Wipe, Eau de Toilette, Eau de Cologne, Parfum, Wachs, inklusive der Darreichungsform als Stift, Roll-On, (Pump-)Spray, Aerosol (schäumend, nicht schäumend oder nach-schäumend), Hautpflegemittel (wie oben beschrieben) als Fußpflegemittel (inklusive Keratolytika, Desodorant), als Insekten abwehrendes Mittel, als Sonnenschutzmittel, als Selbstbräunungsmittel und / oder Aftersun-Präparat, Hautpflegemittel als Rasiermittel oder Aftershave, als Haarentfernungsmittel, als Haarpflegemittel wie z. B. Shampoo (inklusive Shampoo für normales Haar, für schnellfettendes Haar, für trockenes, strapaziertes (geschädigtes) Haar, 2-in-1 Shampoo, Anti-Schuppen-shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (z. B. Gel oder Wachs); Blondiermittel, Haarfärbemittel wie z. B. temporäre, direktziehende, semipermanente Haarfärbemittel, permanente Haarfärbemittel), Hautpflegemittel als dekorative Körperpflegemittel, wie z. B. Nagelpflegemittel (Nagellack und Nagellackentferner), dekorative Kosmetik (z. B. Puder, Lidschatten, Kajalstift, Lippenstift), Hautpflegemittel als Deodorant und / oder Antitranspirant; Mundwasser und Munddusche vorliegen.

Die (insbesondere topischen) kosmetischen oder pharmazeutischen, bevorzugt dermatologischen Zubereitungen können ein, zwei, drei, vier oder mehrere Grund-, Hilfs- und Zusatzstoffe enthalten, wie sie üblicherweise in solchen Zubereitungen verwendet werden, beispielsweise ausgewählt aus der Gruppe bestehend aus Sonnenschutzmittel, Konservierungsmittel, Bakterizide, Fungizide, Viruzide, Kühlwirkstoffe, Insektrepellents (z. B. DEET, IR 3225), Pflanzenextrakte, Pflanzenteile, weitere entzündungshemmende Wirkstoffe, die Wundheilung beschleunigende Stoffe (z. B. Chitin oder Chitosan und dessen Derivate), filmbildende Substanzen (z. B. Polyvinylpyrrolidone oder Chitosan oder dessen Derivate), Antioxidantien, Vitamine, 2-Hydroxycarbonsäuren (z. B. Zitronensäure, Apfelsäure, L-, D- oder dl-Milchsäure), Hautfärbungsmittel (z. B. Walnussextrakte oder Dihydroxyaceton), Wirkstoffe zur Haarwuchsförderung oder Haarwuchshemmung, hautpflegende Mittel (z. B. Cholesterol, Ceramide, Pseudoceramide), weichmachende, anfeuchtende und/oder feuchthaltende Substanzen, Fette, Öle, gesättigte Fettsäuren, ein- oder mehrfach ungesättigte Fettsäuren, α-Hydroxysäuren, Polyhydroxyfettsäuren oder deren Derivate, Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner (z. B. Ethylendiamintetraessigsäure und Derivate), Antischuppenwirkstoffe (z. B. Climbazol, Ketoconazol, Piroctonoleamin, Zink-Pyrithion), Haarpflegemittel, Parfüme, Substanzen zum Verhindern des Schäumens, Farbstoffe, Pigmente, die eine färbende Wirkung haben, Verdickungsmittel (vorteilhaft Siliciumdioxid, Aluminiumsilikate, wie z. B. Bentonite, Polysaccharide bzw. deren Derivate, z. B. Hyaluronsäure, Guarkernmehl, Xanthangummi, Hydroxypropyl-methylcellulose oder Allulose-Derivate, besonders vorteilhaft Polyacrylate wie z. B. Carbopole oder Polyurethane), oberflächenaktive Substanzen und Emulgatoren.

Die jeweils einzusetzenden Mengen an kosmetischen oder pharmazeutischen (gegebenenfalls dermatologischen) Grund-, Hilfs- und Zusatzstoffen und Duftstoffe können in Abhängigkeit von der Art des jeweiligen Produkts vom Fachmann durch einfaches Ausprobieren leicht ermittelt werden.

Erfindungsgemäß umfassen die kosmetischen und pharmazeutischen Zubereitungen sowie Duftstoffkompositionen gegebenenfalls ein, zwei, drei, vier oder mehrere weitere antiinflammatorische wirksame Verbindungen. Es können hierbei alle für kosmetische und/oder dermatologische Anwendungen geeigneten oder gebräuchlichen hautirritations- und/oder hautrötungsverringernden Wirkstoffe verwendet werden. Besonders bevorzugt sind jedoch Kombinationen mit einer, zwei, drei, vier oder mehreren weiteren antiinflammatorisch wirksamen Verbindungen ausgewählt aus der Gruppe der folgenden Verbindungen: steroidale entzündungshemmende Substanzen vom Kortikosteroiden-Typ, bevorzugt Hydrocortison, Hydrocortison-Derivate wie Hydrocortison-17-butyrat, Dexamethason, Dexamethasonphosphat, Methylprednisolon oder Cortison sowie weitere steroidale Entzündungshemmer; nichtsteroidale Entzündungshemmer, bevorzugt Oxicame wie Piroxicam oder Tenoxicam; Salicylate wie Aspirin, Disalcid, Solprin oder Fendosal; Essigsäre-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Benoxaprofen oder Pyrazole wie Phenylbutazon, Oxyphenylbutazon, Febrazon oder Azapropazon; natürliche entzündungshermmende bzw. rötungs- und/oder juckreizlindernde Stoffe, bevorzugt Pflanzenextrakte, spezielle hochwirksame Pflanzenextrakt-Fraktionen sowie aus Pflanzenextrakten isolierte hochreine Wirksubstanzen, besonders bevorzugt sind Extrakte, Fraktionen und Wirksubstanzen aus Kamille, Ingwer, Aloe vera, Commiphora-Arten, Süßholz (Glycyrrhiza-) Arten wie Glycyrrhiza glabra und Glycyrrhiza inflata , Rubia-Arten, Weide, Weidenröschen, Hafer, Calendula, Arnika, Johanniskraut, Geißblatt, Rosmarin, Melisse, Passiflora incarnata, Hamamelis, Pueraria, Dianthus oder Echinacea sowie isolierte Reinsubstanzen hieraus, bevorzugt Bisabolol, Apigenin, Apigenin-7-glucosid, Rosmarinsäure, Boswelliasäure, Phytosterole, Glycyrrhizinsäure, Glabridin, Licochalkon A und Anthranilsäureamide wie insbesondere Avenanthramide oder Dianthramide; natürlich vorkommende Anthranilsäure-Derivate, bevorzugt aus Hafer oder Nelken (Dianthus-Arten); vollsynthetisch oder partialsynthetisch hergestellte Anthranilsäureamide, vorzugsweise Dihydroavenanthramid D (Symrise GmbH / Co. KG); natürlich vorkommende Inhaltsstoffe von Süßholz und/oder vollsynthetische oder partialsynthetisch hergestellte Verbindungen, bevorzugt Tetrahydrolicochalcon A.

Das im Sinne der vorliegenden Erfindung verwendete Bisabolol kann natürlichen oder synthetischen Ursprungs sein, vorzugsweise handelt es sich um "alpha-Bisabolol". Der Begriff "alpha-Bisabolol" umfasst dabei im Rahmen dieses Textes (+)-alpha-Bisabolol, (-)-alpha-Bisabolol, (+)-epi-alpha-Bisabolol und (-)-epi-alpha-Bisabolol sowie Mischungen von zwei, drei oder sämtlichen der genannten Isomeren des alpha-Bisabolol. Insbesondere umfasst der Begriff "alpha-Bisabolol" racemische Gemische von (+/-)-alpha-Bisabolol und/oder (+/-)-epi-alpha-Bisabolol. Vorzugsweise handelt es sich bei dem verwendeten Bisabolol um synthetisch hergestelltes oder um natürliches (-)-alpha-Bisabolol und/oder synthetisches isomerengemischtes alpha-Bisabolol. Sofern natürliches (-)-alpha-Bisabolol verwendet wird, kann dieses auch als Bestandteil eines ätherischen Öls oder eines Pflanzenextraktes oder einer Fraktion davon eingesetzt werden, beispielsweise als Bestandteil von (Fraktionen von) Ölen oder Extrakten der Kamille oder von Vanillosmopsis (insbesondere Vanillosmopsis erythropappa oder Vanillosmopsis arborea). Synthetisches alpha-Bisabolol ist beispielsweise unter dem Namen "Dragosantol" von Symrise GmbH & Co. KG erhältlich.

Im Falle von Ingwer-Extrakt handelt es sich vorzugsweise um durch Extraktion mit Methanol, Ethanol, iso-Propanol, Aceton, Ethylacetat, Kohlendioxid (CO₂), Hexan, Dichlormethan, Chloroform oder anderen Lösungsmitteln oder Lösungsmittelgemischen vergleichbarer Polarität hergestellte Extrakte der frischen oder getrockneten Ingwerwurzel. Die Extrakte sind durch das Vorhandensein wirksamer hautirritationsverringernder Mengen an Inhaltsstoffen wie z. B. Gingerolen, Shogaolen, Gingerdiolen, Dehydrogingerdionen und/oder Paradolen gekennzeichnet.

Die Menge der einen, zwei, drei, vier oder mehreren weiteren antiinflammatorisch wirkenden Verbindungen (Antiirritantien) in den erfindungsgemäßen Zubereitungen beträgt vorzugsweise insgesamt 0,0001 bis 20 Gew.-%, besonders bevorzugt 0,0001 - 10 Gew.-%, insbesondere 0,001 - 5 Gew.-%, bezogen auf das Gesamtgewicht der Zubereitung.

Erfindungsgemäße kosmetische und pharmazeutische, bevorzugt dermatologische Zubereitungen können des weiteren auch ein, zwei, drei, vier oder mehrere Antioxidantien enthalten, wobei alle für kosmetische und pharmazeutische, bevorzugt dermatologische Anwendungen geeigneten oder gebräuchlichen Antioxidantien verwendet werden können. Vorteilhaft werden die ein, zwei, drei, vier oder mehrere Antioxidantien gewählt aus der Gruppe bestehend aus Aminosäuren (z. B. Glycin, Histidin, Tyrosin, Tryptophan) und deren Derivaten, Imidazolen (z. B. Urocaninsäure) und deren Derivaten, Peptiden wie D, L-Carnosin, D-Carnosin, L-Carnosin und deren Derivaten (z. B. Anserin), Carotinoiden, Carotinen (z. B. α-Carotin, β-Carotin, Lycopin) und deren Derivaten, Chlorogensäure und deren Derivate, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze), (Metall)-Chelatoren, z. B. α-Hydroxyfettsäuren, Palmitinsäure, Phytinsäure, Lactoferrin, α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat, Ascorbyl glycoside wie z. B. 6-O-Acyl-2-O-α-D-glucopyranosyl-L-Ascorbinsäure, 6-O-Acyl-2-O-β-D-glucopyranosyl-L-Ascorbinsäure, 2-O-α-D-glucopyranosyl-L-Ascorbinsäure oder 2-O-β-D-glucopyranosyl-L-Ascorbinsäure), Tocopherole und deren Derivate (z.B. Vitamin-Vitamin-E-acetat), Vitamin A und dessen Derivate (Vitamin-A-palmitat) sowie Coniferylbenzoat des Benzoeharzes, Rutinsäure und deren Derivate, α-Glucosylrutin, Quercetin und dessen Derivate, Rosmarinsäure, Carnosol, Carnosolsäure, Resveratrol, Kaffeesäure und deren Derivate, Sinapinsäure und deren Derivate, Ferulasäure und deren Derivate, Furfurylidenglucitol, Curcuminoide, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z. B. ZnO, ZnSO4) Selen und dessen Derivate (z. B. Selenmethionin), Stilbene und deren Derivate (z. B. Stilbenoxid, trans-Stilbenoxid) sowie Derivate (Salze, Ester, Ether, Zucker, Nukleotide, Nukleoside, Peptide und Lipide) dieser genannten Wirkstoffe oder antioxidativ wirksame Extrakte oder Fraktionen von Pflanzen wie z. B. Grüntee, Rooibos, Honeybush, Traube, Rosmarin, Salbei, Melisse, Thymian, Lavendel, Olive, Hafer, Kakao, Ginkgo, Ginseng, Süßholz, Geißblatt, Sophora, Pueraria, Pinus, Citrus, Phyllanthus emblica oder Johanniskraut.

Die Gesamtmenge der Antioxidantien (eine oder mehrere Verbindungen) in den erfindungsgemäßen Zubereitungen beträgt vorzugsweise 0,01 bis 20 Gew.%, besonders bevorzugt 0,05 - 10 Gew.%, insbesondere 0,2 - 5 Gew.%, bezogen auf das Gesamtgewicht der Zubereitung.

Vorteilhaft enthalten erfindungsgemäße kosmetische und pharmazeutische, bevorzugt dermatologische Zubereitungen auch einen, zwei, drei, vier oder mehrere UV-Filter, bevorzugt ausgewählt aus der Gruppe der UVA-Filter, UVB-Filter und/oder anorganischer Pigmente. Die erfindungsgemäßen Zubereitungen können dabei in verschiedenen Formulierungen vorliegen, wie sie üblicherweise für Sonnenschutzzubereitungen zum Schutz von Haut und Haar gegen ultraviolette Strahlung eingesetzt werden. So können sie bevorzugt eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol bilden. Dabei beträgt die Gesamtmenge der UV-Filtersubstanzen von 0,01 Gew.-% bis 40 Gew.-%, vorzugsweise 0,1% bis 10 Gew.-%, insbesondere 1,0 bis 5,0 Gew.-%, bezogen auf das Gesamtgewicht der Zubereitungen.

Vorteilhafte UV-Filter sind z.B.: p-Aminobenzoesäure, p-Aminobenzoesäureethylester (25 Mol) ethoxyliert, p-Dimethylaminobenzoesäure-2-ethylhexylester, p-Aminobenzoesäureethylester (2 Mol) N-propoxyliert, p-Aminobenzoesäureglycerinester, Salicylsäure-homomenthylester (Homosalate) (Neo Heliopan^{®}HMS), Salicylsäure-2-ethylhexylester (Neo Heliopan^{®}OS), Triethanolaminsalicylat, 4-Isopropylbenzylsalicylat, Anthranilsäurementhylester (Neo Heliopan^{®}MA), Diisopropylzimtsäureethylester, p-Methoxyzimtsäure-2-ethylhexylester (Neo Heliopan^{®}AV), Diisopropylzimtsäuremethylester, p-Methoxyzimtsäureisoamylester (Neo Heliopan^{®}E 1000), p-Methoxyzimtsäure-diethanolaminsalz, p-Methoxyzimtsäureisopropylester, 2-Ethylhexyl-2-cyano-3,3-diphenylacrylat (Neo Heliopan^{®}303), Ethyl-2-cyano-3,3'-diphenylacrylat, 2-Phenylbenzimidazolsulfonsäure und Salze (Neo Heliopan^{®}Hydro), 3-(4'-Trimethylammonium)-benzyliden-bornan-2-on-methylsulfat, Terephthalyliden-dibornansulfonsäure und Salze (Mexoryl^{®}SX), 4-t-Butyl-4'-methoxydibenzoylmethan (Avobenzon) / (Neo Heliopan^{®}357), β-Imidazol-4(5)-acrylsäure (Urocaninsäure), 2-Hydroxy-4-methoxybenzophenon (Neo Heliopan^{®}BB), 2-Hydroxy-4-methoxybenzophenon-5-sulfonsäure, Dihydroxy-4-methoxybenzophenon, 2,4-Dihydroxybenzophenon, Tetrahydroxybenzophenon, 2,2'-Dihydroxy-4,4'-dimethoxybenzophenon, 2-Hydroxy-4-n-octoxybenzophenon, 2-Hydroxy-4-methoxy-4'-methylbenzophenon, 3-(4'-Sulfo)benzyliden-bornan-2-on und Salze, 3-(4'-Methylbenzyliden)-d,l-campher (Neo Heliopan^{®}MBC), 3-Benzyliden-d,1-campher, 4-Isopropyldibenzoylmethan, 2,4,6-Trianilino-(p-carbo-2'-ethylhexyl-1'-oxy)-1,3,5-triazin, Phenylen-bis-benzimidazyl-tetrasulfonsäure-dinatriumsalz (Neo Heliopan^{®}AP), 2,2'-(1,4-Phenylen)-bis-(1H-benzimidazol-4,6-disulfonsäure), Mononatriumsalz, N-[(2 und 4)-[2-(oxoborn-3-yliden)methyl]benzyl]-acrylamid-Polymer, Phenol, -(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL), 4,4'-[(6-[4-(1,1-Dimethyl)-aminocarbonyl)-phenylamino]-1,3,5-triazin-2,4-diyl)diimino]-bis-(benzoesäure-2-ethylhexylester) (Uvasorb^{®}HEB), 2,2'-Methylen-bis-(6-(2H-benztriazol-2-yl)-4-1,1,3,3-tetramethylbutyl)-phenol), (Tinosorb^{®}M), 2,4-bis-[4-(2-ethylhexyloxy)-2-hydroxyphenyl]-1,3,5-triazin, Benzylidenmalonat-Polysiloxan (Parsol^{®}SLX), Glyceryl-ethylhexanoat-dimethoxycinnamat, Dinatrium-2,2'-dihydroxy-4,4'-dimethoxy-5,5'-disulfo-benzophenon, Dipropylenglykolsalicylat, Natrium-hydroxymethoxybenzophenon-sulfonat, 4,4',4-(1,3,5-Triazin-2,4,6-triyltriimino)-tris-benzoesäure-tris(2-ethylhexylester) (Uvinul^{®}T150), 2,4-Bis-[{(4-(2-Ethyl-hexyloxy)-2-hydroxy}-phenyl]-6-(4-methoxyphenyl)-1,3,5-triazin, (Tinosorb^{®}S), 2,4-Bis-[{(4-(3-sulfonato)-2-hydroxy-propyloxy)-2-hydroxy}-phenyl]-6-(4-methoxyphenyl)-1,3,5-triazin-Natriumsalz, 2,4-Bis-[{(3-(2-Propyloxy)-2-hydroxy-propyloxy)-2-hydroxy}-phenyl]-6-(4-methoxy-phenyl)-1,3,5-triazin, 2,4-Bis-[{4-(2-Ethyl-hexyloxy)-2-hydroxy}-phenyl]-6-[4-(2-methoxyethyl-carbonyl)-phenylamino]-1,3,5-triazin, 2,4-Bis-[{4-(3-(2-propyloxy)-2-hydroxy-propyloxy)-2-hydroxy}-phenyl]-6-[4-(2-ethylcarboxyl)-phenylamino]-1,3,5-triazin, 2,4-Bis-[{4-(2-Ethyl-hexyloxy)-2-hydroxy}-phenyl]-6-(1-methyl-pyrrol-2-yl-)-1,3,5-triazin, 2,4-Bis-[{4-tris-(trimethylsiloxy-silylpropyloxy)-2-hydroxy}-phenyl]-6-(4-methoxyphenyl)-1,3,5-triazin, 2,4-Bis-[{4-(2"-Methylpropenyloxy)-2-hydroxy}-phenyl]-6-(4-methoxyphenyl)-1,3,5-triazin, 2,4-Bis-[{4-(1',1',1',3',5',5',5'-Heptamethylsiloxy-2"-methyl-propyloxy)-2-hydroxy}-phenyl]-6-(4-methoxyphenyl)-1,3,5-triazin, 2-(4-Diethylamino-2-hydroxybenzoyl)-benzoesäure-hexylester (Uvinul^{®} A Plus), Indanylidenverbindungen gemäß DE 100 55 940 (= WO 02/38537).

Besonders zur Kombination geeignete UV-Absorber werden ausgewählt aus der Gruppe bestehend aus p-Aminobenzoesäure, 3-(4'-Trimethylammonium)-benzyliden-bornan-2-on-methylsulfat, Salicylsäure-homomenthylester (Neo Heliopan^{®}HMS), 2-Hydroxy-4-methoxy-benzophenon (Neo Heliopan^{®}BB), 2-Phenylbenzimidazolsulfonsäure (Neo Heliopan^{®}Hydro), Terephthalylidendibornansulfonsäure und Salze (Mexoryl^{®}SX), 4-tert.-Butyl-4'-methoxydibenzoylmethan (Neo Heliopan^{®}357), 3-(4'-Sulfo)benzyliden-bornan-2-on und Salze, 2-Ethylhexyl-2-cyano-3,3-diphenylacrylat (Neo Heliopan^{®}303), N-[(2 und 4)-[2-(oxoborn-3-yliden)methyl]benzyl]-acrylamid-Polymer, p-Methoxyzimtsäure-2-ethylhexylester (Neo Heliopan^{®}AV), p-Aminobenzoesäure-ethylester (25 Mol) ethoxyliert, p-Methoxyzimtsäure-isoamylester (Neo Heliopan^{®}E1000), 2,4,6-Trianilino-(p-carbo-2'-ethylhexyl-1'-oxy)-1,3,5-triazin (Uvinul^{®}T150), Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL), 4,4'-[(6-[4-(1,1-Dimethyl)-aminocarbonyl)-phenylamino]-1,3,5-triazin-2,4-diyl)-diimino]-bis-(benzoesäure-2-ethylhexylester), (UvasorbHEB), 3-(4'-Methylbenzyliden)-d,1-campher (Neo Helipan^{®}MBC), 3-Benzylidencampher, Salicylsäure-2-ethylhexylester (Neo Helipan^{®}OS), 4-Dimethylaminobenzoesäure-2-ethylhexylester (Padimate O), Hydroxy-4-methoxy-benzophenon-5-sulfonsäure und Na-Salz, 2,2'-Methylen-bis-(6-(2H-benztriazol-2- yl)-4-1,1,3,3-tetramethylbutyl)-phenol), (Tinosorb^{®}M), Phenylen-bis-benzimidazyl-tetrasulfonsäure-dinatriumsalz (Neo Heliopan^{®}AP), 2,4-Bis-[{(4-(2-Ethyl-hexyloxy)-2-hydroxy}-phenyl]-6-(4-methoxyphenyl)-1,3,5-triazin, (Tinosorb^{®}S), Benzylidenmalonat-Polysiloxan (Parsol^{®}SLX), Menthylanthranilat (Neo Heliopan^{®}MA), 2-(4-Diethylamino-2-hydroxybenzoyl)-benzoesäure-hexylester (Uvinul^{®} A Plus), Indanylidenverbindungen gemäß DE 100 55 940 (= WO 02/38537).

Vorteilhafte anorganische Lichtschutzpigmente werden ausgewählt aus der Gruppe bestehend aus feindisperse Metalloxide und Metallsalze, vorzugsweise Titandioxide, Zinkoxid (ZnO), Eisenoxide (z.B. Fe₂O₃), Aluminiumoxid (Al₂O₃); Ceroxide (z. B. Ce₂O₃), Manganoxide (z. B. MnO), Zirkoniumoxid (ZrO₂), Siliciumoxid (SiO₂), Mischoxide der entsprechenden Metalle sowie Abmischungen aus solchen Oxiden, Bariumsulfat und Zinkstearat. Besonders bevorzugt handelt es sich um Pigmente auf der Basis von TiO₂ oder Zinkoxid. Die Partikel besitzen in bevorzugten Ausführungsformen einen mittleren Durchmesser von weniger als 100 nm, vorzugsweise ziwschen 5 und 50 nm und insbesondere bevorzugt ziwschen 15 und 30 nm. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d. h. hydrophilisiert oder hydrophobisiert vorliegen. Typische Beispiele sind gecoatete Titandioxide, wie z. B. Titandioxid T 805 (Degussa) oder Eusolex^{®} T2000 (Merck) oder gecoatetes Zinkoxid, wie z. B. Zinc Oxid NDM. Als hydrophobe Coatingmittel kommen dabei vor allem Silicone und dabei speziell Trialkoxyoctysilane oder Simethicone in Frage. In Sonnenschutzmitteln werden bevorzugt sogenannte Mikro- oder Nanopigmente eingesetzt. Vorzugsweise werden Zink-Mikro- oder -Nanopigmente eingesetzt.

Die Gesamtmenge an anorganischen Pigmenten, insbesondere hydrophoben anorganischen Mikropigmenten in den erfindungsgemäßen kosmetischen oder pharmazeutischen, bevorzugt dermatologischen Zubereitungen liegt vorzugsweise im Bereich von 0,1 bis 30 Gew.-%, bevorzugt 0,1 bis 10,0, insbesondere 0.5 bis 6.0 Gew.-%, bezogen auf das Gesamtgewicht der erfindungsgemäßen kosmetischen und pharmazeutischen, bevorzugt dermatologischen Zubereitungen.

Erfindungsgemäße kosmetische und pharmazeutische, bevorzugt dermatologische Zubereitungen können auch ein, zwei, drei, vier oder mehrere Wirkstoffe und Wirkstoffkombinationen gegen Hautalterung und Falten enthalten. Erfindungsgemäß können hierbei alle für kosmetische und pharmazeutische, bevorzugt dermatologische Anwendungen geeigneten oder gebräuchlichen Wirkstoffe gegen Hautalterung und Falten verwendet werden. Vorteilhafte Wirkstoffe gegen Hautalterung und Falten werden ausgewählt aus der Gruppe bestehend aus Sojaprotein bzw. Proteinhydrolysate, Sojaisoflavone, hydrolysiertes Reisprotein, hydrolysiertes Haselnußprotein, Oligopeptide aus hydrolysiertem Hibiscus esculentus Extrakt, Weizenprotein, β-Glucane z. B. aus Hafer und deren Derivate, Glycoproteine, Ursolsäure und ihre Salze, Betulin, Betulinsäure und ihre Salze, Retinol, Retinolpalmitat, Propylgallat, Precocenen, 6-Hydroxy-7-methoxy-2,2-dimethyl-1(2H)-benzopyran, 3,4-Dihydro-6-hydroxy-7-methoxy-2,2-dimethyl-1(2H)-benzopyran, Creatin oder andere synthetische oder natürliche Wirkstoffe gegen Hautalterung und Falten, wobei letztere auch in Form eines Extrakts aus Pflanzen, wie z. B. Grüntee, Rubus fruticosus, Sanguisorba officinalis, Centella asiatica, Ribes nigrum, Passiflora incarnata, Filipendula ulmaria, Phyllanthus emblica, Potentilla-Arten, Okra, Algen, Nachtkerze, Granatpafel, Frauenmantel, Rosmarin, Salbei, Echinacea, Birke, Apfel oder Soja verwendet werden können.

Besonders bevorzugt zur Verwendung als ein, zwei, drei, vier oder mehrere weitere Wirkstoffe gegen Hautalterung sind β-Glucan, insbesondere bevorzugt ist 1,3-1,4-verknüpftes β-Glucan aus Hafer, Rubus fruticosus Extrakt oder Weizenprotein.

Erfindungsgemäße kosmetische und pharmazeutische, bevorzugt dermatologische Zubereitungen können auch ein, zwei, drei, vier oder mehrere Wirkstoffe enthalten, die die Haut- und Haartönung bzw. -bräunung auf chemische oder natürliche Weise stimulieren. Insbesondere bevorzugt sind dabei Substrate oder Substratanaloga der Tyrosinase wie L-Tyrosin, L-DOPA oder L-Dihydroxyphenylalanin, Stimulatoren der Tyrosinase-Aktivität oder -Expression wie Theophyllin, Kaffein, Proopiomelanocortin-Peptide wie ACTH, alpha-MSH, deren Peptidanaloga und andere Substanzen, die an den Melanocortin-Rezeptor binden, Peptide wie Val-Gly-Val-Ala-Pro-Gly, Lys-Ile-Gly-Arg-Lys oder Leu-lle-Gly-Lys, Purine, Pyrimidine, Folsäure, Kupfer-Salze wie Kupfergluconat, -chlorid oder -pyrrolidonat, Flavonoide, Flavanon-Gylcoside wie Naringin und Hesperidin, Melanin-Deraivate wie Melasyn-100 und MelanZe, Diacylglyerole, aliphatische oder cyclische Diole, Psoralene, Prostaglandine und deren Analoga, Aktivatoren der Adenylatcyclase sowie Verbindungen, die den Transfer von Melanosomen in Keratinozyten aktivieren wie Serinproteasen oder Agonisten des Rezeptors PAR-2, Extrakte aus Pflanzen und Pflanzenteilen der Chrysanthemum-Art, Sanguisorba-Art, Walnussextrakte, Urucumextrakte, Rhabarberextrakte, Erytrulose und Dihydroxyaceton.

Erfindungsgemäße kosmetische und pharmazeutische, bevorzugt dermatologische Zubereitungen können auch in Kombination mit ein, zwei, drei, vier oder mehreren hautaufhellenden Wirkstoffen eingesetzt werden. Erfindungsgemäß können hierbei alle für kosmetische und pharmazeutische, bevorzugt dermatologische Anwendungen geeigneten oder gebräuchlichen hautaufhellenden Wirkstoffe verwendet werden. Vorteilhafte ein, zwei, drei, vier oder mehreren hautaufhellenden Wirkstoffe werden ausgewählt aus der Gruppe bestehend aus Kojisäure (5-Hydroxy-2-hydroxymethyl-4-pyranon), Kojisäurederivate wie z. B. Kojisäuredipalmitat, Arbutin, Ascorbinsäure, Ascorbinsäurederivate, Hydrochinon, Hydrochinonderivate, Resorcin, schwefelhaltige Moleküle wie z. B. Cystein, alpha-Hydroxysäuren, (z. B. Zitronensäure, Milchsäure, Apfelsäure) und deren Derivate, N-Acetyl-Tyrosin und -Derivate, Undecenoylphenylalanin, Gluconsäure, 4-Alkylresorcine, 4-(1-Phenylethyl)1,3-benzenediol, Chromon-Derivate wie Aloesin, Flavonoide, Thymolderivate, 1-Aminoethylphosphinsäure, Thioharnstoffderivate, Ellagsäure, Nicotinamid, Zinksalze wie z. B. Zinkchlorid oder - gluconat, Thujaplicin und -Derivate, Triterpene wie Maslinsäure, Sterole wie Ergosterol, Benzofuranone wie Senkyunolid, Vinyl- und Ethylgujacol, Inhibitoren der Stickstoffoxid-Synthese wie z. B., L-Nitroarginin und dessen Derivate, 2,7-Dinitroindazol oder Thiocitrullin, Metallchelatoren (z. B. α-Hydroxyfettsäuren, Palmitinsäure, Phytinsäure, Lactoferrin, Huminsäure, Gallensäure, Gallenextrakte, Bilirubin, Biliverdin, EDTA, EGTA und deren Derivate), Retinoide, Sojamilch, Serin-Protease-Inhibitoren oder Liponsäure oder andere synthetische oder natürliche Wirkstoffe zur Haut- und Haaraufhellung, wobei letztere auch in Form eines Extrakts aus Pflanzen, wie z. B. Bearberry-Extrakt, Reis-Extrakt, Süßholzwurzel-Extrakt oder daraus angereicherte Bestandteile wie Glabridin oder Licochalkon A, Artocarpus-Extrakt, Extrakt von Rumex- und Ramulus-Arten, Extrakte aus Kieferarten (Pinus) und Extrakte aus Vitis-Arten oder daraus angereicherte Stilbenderivate, Extrakt aus Saxifraga, Maulbeer, Scutelleria oder/und Trauben verwendet werden.

Erfindungsgemäße kosmetische und pharmazeutische, bevorzugt dermatologische Zubereitungen können vorteilhafterweise auch ein, zwei, drei, vier oder mehrere Feuchthalteregulatoren enthalten. Als Feuchthalteregulatoren ("moisturizer") finden vorzugsweise ein, zwei, drei, vier oder mehrere der folgenden Stoffe Verwendung: Natriumlactat, Harnstoff, -derivate, Alkohole, Glycerin, Diole wie Propylenglykol, 1,2-Pentandiol, 1,2-Hexandiol und 1,2-Octandiol sowie Mischungen enthaltend mindestens zwei Diole, Kollagen, Elastin oder Hyaluronsäure, Diacyladipate, Petrolatum, Urocaninsäure, Lecithin, Panthenol, Phytantriol, Lycopen, (Pseudo-)Ceramide, Glykosphingolipide, Cholesterol, Phytosterole, Chitosan, Chondroitinsulfat, Lanolin, Lanolinester, Aminosäuren, alpha-Hydroxysäuren (z.B. Zitronensäure, Milchsäure, Äpfelsäure) und deren Derivate, Mono-, Di- und Oligosaccharide wie beispielsweise Glucose, Galactose, Fructose, Mannose, Fruchtzucker und Lactose, Polyzucker wie β-Glucane, insbesondere 1,3-1,4-β-Glucan aus Hafer, alpha-Hydroxy-Fettsäuren, Triterpensäuren wie Betulinsäure oder Ursolsäure und Algenextrakte.

Erfindungsgemäße kosmetische und pharmazeutische, bevorzugt dermatologische Zubereitungen können auch zusammen mit ein, zwei, drei, vier oder mehreren Osmolyten eingesetzt werden. Als Osmolyte werden vorzugsweise ein, zwei, drei, vier oder mehrere der folgenden Verbindungen ausgewählt: Substanzen aus der Gruppe der Zuckeralkohole (myo-Inositol, Mannitol, Sorbitol), quaternäre Amine wie Taurin, Cholin, Betain, Betain-Glycin, Ectoin, Diglycerolphosphat, Phosphorylcholin, Glycerophosphorylcholine, Aminosäuren wie Glutamin, Glycin, Alanin, Glutamat, Aspartat oder Prolin, Phosphatidylcholin, Phosphatidylinositol, anorganische Phosphate, sowie Polymere der genannten Verbindungen wie Proteine, Peptide, Polyaminosäuren und Polyole. Alle Osmolyte haben zugleich eine hautbefeuchtende Wirkung.

Erfindungsgemäße kosmetische und pharmazeutische, bevorzugt dermatologische Zubereitungen können vorteilhafterweise auch ein, zwei, drei, vier oder mehrere Vitamine und Vitaminvorstufen enthalten, wobei alle für kosmetische und pharmazeutische, bevorzugt dermatologische Anwendungen geeigneten oder gebräuchlichen Vitamine und Vitaminvorstufen verwendet werden können. Als Vitamine und Vitaminvorstufen seien vorzugsweise genannt: Vitamin A (Retinol) und seine Derivate (z. B. Vitamin A Acetat, Vitamin A Säure, Vitamin A Aldehyd, Vitamin A Palmitat, Vitamin A Propionat), Vitamin B1 (Thiamin) und seine Salze (z. B. Vitamin B1 Hydrochlorid, Vitamin B1 Mononitrat, Thiamin Diphosphat, Thiamin Pyrophosphat), Vitamin B12 (Cobalamin), Vitamin B2 (Vitamin G, Riboflavin) und seine Derivate (z.B. Vitamin B2 Tetraacetate), Vitamin B3 und seine Derivate (z.B. Nikotinamid Ascorbat, Nikotinamid Glycolat, Nikotinamid Hydroxycitrat, Nikotinamid Lactat, Nikotinamid Malat, Nikotinamid Mandelat, Nikotinamid Salicylat, Nikotinamid Thioctat), Vitamin B4 (Adenin) und seine Derivate (z. B. Adenine Riboside, Dinatrium Flavin Adenin Dinucleotid, Nikotinamid Adenin Dinucleotid), Provitamin B5, Vitamin B5 (Pantothensäure) und seine Derivate (z.B. Acetyl Pantothenyl Ethyl Ether, Allantoin Calcium Pantothenat, Allantoin DL-Pantothenylalcohol, Bis(Pantothenamidoethyl) Disulfid, Calcium Pantothenat, Hydroxyethyl Pantothenamid MEA, Natrium Pantothenat, N-D-Pantothenoyl-2-(2-Aminoethoxy)Ethanol, N-D-Pantothenoyl-2-Aminoethanol, N-Hydroxyethoxyethyl Pantothenamid, N-Hydroxyethyl Pantothenamide, Pantothenamid MEA, Pantothenol, Pantothensäure Lactone, Pantothensäure Polypeptid, Pantothenyl Ethylether), Vitamin B6 (Pyridoxol, Pyroxidal, Pyridoxamin) und seine Derivate (z.B. Pyridoxin Dicaprylat, Vitamin B6 Dilaurate, Vitamin B6 Dioctanoate, Vitamin B6 Dipalmitate, Pyridoxin Glycyrrhetinat, Vitamin B6 Hydrochloride, Vitamin B6 Phosphate, Vitamin B6 Serine, Vitamin B6 Tripalmitate), Vitamin C (Ascorbinsäure) und seine Derivate (z.B. 3-O-Ethyl Ascorbinsäure, Allantoin Ascorbat, Aminopropyl Ascorbyl Phosphat, Araboascorbinsäure, Mononatriumsalz, Ascorbinsäure Palmitat, Ascorbinsäure Polypeptid, Ascorbosilane C, Ascorbyl Dipalmitat, Ascorbyl Glucosid, Ascorbyl Inositol Nikotinat, Ascorbyl Linoleat, Ascorbyl Methylsilanol Pectinat, Ascorbyl Nikotinamid, Ascorbyl Phosphat Magnesium, Ascorbyl Stearat, Ascorbyl Tetraisopalmitat, Ascorbyl Tocopheryl Maleat, Calcium Ascorbat, Chitosan Ascorbat, D-Arabino-Ascorbinsäure, Dinatrium Ascorbyl Sulfate, Glucosamine Ascorbat, Inositol Hexanikotinat Hexaascorbat, Isoascorbinsäure, L-Ascorbinsäure, 2-(Dihydrogen Phosphat), Trinatriumsalz, L-Ascorbinsäure, 2-[(3-Cholest-5-en-3-yl Hydrogen Phosphat], Mononatriumsalz, L-Ascorbinsäure, 2-O--D-Glucopyranosyl-, L-Ascorbinsäure, 3-O-Ethyl Ether, Magnesium Ascorbat, Magnesium Ascorbylborat, Methoxy PEG-7 Ascorbinsäure, Methylsilanol Ascorbat, Kalium Ascorbyl Tocopheryl Phosphat, Kalium Ascorbylborate, Natrium Ascorbat, Natrium Ascorbyl Phosphat, Natrium Ascorbyl/Cholesteryl Phosphat, Natrium IsoAscorbat, Natrium L-Ascorbyl-2-Phosphat, Tetrahexyldecyl Ascorbat), Provitamin D, Vitamin D (Calciol) und seine Derivate (z. B. Vitamin D2, Vitamin D3), Vitamin E (D-Alpha-Tocopherol) und seine Derivate (z. B. dl-alpha-Tocopherol, Polyoxypropylene / Polyoxyethylene / Tocopherol Ether, Polypropylene Glycol / Tocopherol Ether, Tocopherol Cysteamin, Tocopherol Phosphat, Natrium Vitamin E Phosphat, Vitamin E Acetat, Vitamin E Linoleate, Vitamin E Nicotinate, Vitamin E Succinate), Vitamin F (Essentielle Fettsäuren, Linolensäure und Linolsäure) und seine Derivate (z.B. Vitamin F Ethyl Ester, Vitamin F Glyceryl Ester), Vitamin H (Vitamin B7, Biotin), Vitamin K1 (Phylloquinone, Phytonadione) und Vitamin K3 (Menadione, Menachinon).

Erfindungsgemäße kosmetische und pharmazeutische, bevorzugt dermatologische Zubereitungen können ebenfalls einen, zwei, drei, vier oder mehrere weitere Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden. Hinsichtlich der verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt beginnenden Tabelle aufgeführt sind. Vorteilhaft sind insbesondere die Extrakte aus Aloe, Algen, Apfel, Aprikose, Arnika, Avocado, Birke, Brennnessel, Brombeer, Calendula, Efeu, Eibisch, Eichenrinde, Erdbeer, Fichte, Geißblatt, Gerste, Ginkgo, Ginseng, Granatapfel, Grapefruit, Gurke, Hafer, Hamamelis, Hauhechel, Henna, Himbeer, Holunder, Honeybusch, Hopfen, Huflattich, Kiwi, Klette, Kokosnuss, Lavendel, Limette, Linde, Malve, Mandel, Mango, Mäusedorn, Melisse, Olive, Orange, Pfefferminze, Pueraria, Quendel, Rooibos, Rose, Rosmarin, Rosskastanie, Salbei, Sandelholz, Schafgarbe, Schachtelhalm, Sophora, Süßholz, Taubnessel, Tee (grün, weiß, schwarz), Thymian, Traube, Wacholder, Weide, Weidenröschen, Weißdorn, Weizen, Wiesenschaumkraut, Zimt, Zitrone und Zitronengras. Besonders bevorzugt sind dabei die Extrakte aus Aloe vera, Algen, Arnika, Brennnessel, Calendula, Hamamelis, Linde, Ginseng, Gurke, Rosmarin und Salbei. Es können auch Mischungen aus zwei oder mehreren Pflanzenextrakten eingesetzt werden. Als Extraktionsmittel zur Herstellung der genannten Pflanzenextrakte können u.a. Wasser, Alkohole sowie deren Mischungen verwendet werden. Unter den Alkoholen sind dabei niedere Alkohole wie Ethanol und Isopropanol, aber auch mehrwertige Alkohole wie Ethylenglykol, Propylenglykol und Butylenglykol bevorzugt, und zwar sowohl als alleiniges Extraktionsmitttel als auch in Mischungen mit Wasser. Die Pflanzenextrakte können sowohl in reiner als auch in verdünnter Form eingesetzt werden.

Erfindungsgemäße kosmetische und pharmazeutische, bevorzugt dermatologische Zubereitungen können vorteilhaft auch ein, zwei, drei, vier oder mehrere Kühlmittel enthalten. Als Kühlmittel seien vorzugsweise genannt: I-Menthol, d-Menthol, racemisches Menthol, Menthonglycerinacetal, Menthyllactat, substituierte Menthyl-3-carbonsäureamide (z. B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z. B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Darüber hinaus können die erfindungsgemäßen kosmetischen und pharmazeutischen, bevorzugt dermatologischen Zubereitungen auch ein, zwei, drei, vier oder mehrere schweißhemmende Wirkstoffe (Antitranspirantien) und/oder Geruchsabsorber enthalten. Als schweißhemmende Wirkstoffe kommen vorzugsweise Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Für die Anwendung in kosmetischen und pharmazeutischen, bevorzugt dermatologischen Antitranspirantien haben sich vor allem die Aluminiumsalze und - in etwas geringerem Maße - Aluminium/Zirkoniumsalz-Kombinationen bewährt. Daneben erwähnenswert sind die teilneutralisierten und damit besser hautverträglichen, aber nicht ganz so wirksamen Aluminiumhydroxychloride. Neben Aluminiumsalzen kommen auch weitere Substanzen in Betracht wie zum Beispiel a) eiweißfällende Substanzen wie u. a. Formaldehyd, Glutaraldehyd, natürliche und synthetische Gerbstoffe sowie Trichloressigsäure, die einen oberflächlichen Verschluß der Schweißdrüsen herbeiführen b) Lokalanästhetika (u.a. verdünnte Lösungen von z. B. Lidokain, Prilokain oder Gemischen derartiger Substanzen), die durch Blockade der peripheren Nervenbahnen die sympathische Versorgung der Schweißdrüsen ausschalten, c) Zeolithe vom X-, A- oder Y-Typ, die neben der Reduktion der Schweißsekretion auch als Adsorbentien für schlechte Gerüche fungieren und d) Botulinustoxin (Toxin des Bakteriums *Chlostridium botulinum),* welches auch bei Hyperhidrose, einer krankhaft erhöhten Schweißsekretion, zum Einsatz gelangt und dessen Wirkung auf einer irreversiblen Blockierung der Freisetzung der für die Schweißsekretion relevanten Transmittersubstanz Acetylcholin beruht.

Geruchsabsorber sind beispielsweise die in DE 40 09 347 beschriebenen Schichtsilikate, von diesen insbesondere Montmorillonit, Kaolinit, Nontronit, Saponit, Hectorit, Bentonit, Smectit, ferner beispielsweise Zinksalze der Ricinolsäure. Dazu zählen ebenfalls Deodorantien, bakterizide bzw. bakteriostatische deodoriende Substanzen, wie z. B. Hexachlorophen, 2,4,4'-Trichlor-2'hydroxydiphenylether (Irgasan), 1,6-Di-(4-chlorphenylbiguanido)-hexan (Chlorhexidin), 3,4,4'-Trichlorcarbanilid, sowie die in DE 37 40 186, DE 39 38 140, DE 42 04 321, DE 42 29 707, DE 42 29 737, DE 42 37 081, DE 43 09 372, DE 43 24 219 beschriebenen wirksamen Agenzien, und kationaktive Substanzen enthalten, wie z. B. quaternäre Ammoniumsalze und Geruchsabsorber, wie z. B. Grillocin^{®} (Kombination von Zinkrizinoleat und verschiedenen Zusätzen) oder Triethylcitrat, gegebenenfalls in Kombination mit Ionenaustauschharzen.

In verschiedenen Fällen kann es auch vorteilhaft sein den erfindungsgemäßen kosmetischen und pharmazeutischen, bevorzugt dermatologischen Zubereitungen ein, zwei, drei, vier oder mehrere Substanzen zuzusetzen, die vornehmlich zur Inhibition des Wachstums unerwünschter Mikroorganismen auf oder in tierischen Organismen eingesetzt werden. Erwähnenswert sind insoweit neben klassischen Konservierungsmitteln als weitere Wirkstoffe neben der großen Gruppe der klassischen Antibiotika insbesondere die für Kosmetika relevanten Produkte wie Triclosan, Climbazol, Zinkpyrithion, Ichthyol, Octopirox (1-Hydroxy-4-methyl-6-(2,4,4-trimethylpentyl)-2(1 H)-pyridone, 2-aminoethanol), Chitosan, Farnesol, Octoxyglycerin, Glycerinmonolaurat, Arylalkylakohole wie z. B. Phenylethylalkohol, 3-Phenyl-1-propanol, Vetikol oder Muguetalkohol, Polyglycerolester wie z.B. Polyglyceryl-3-caprylate sowie aliphatische Diole wie z. B. 1,2-Decandiol oder Kombinationen der genannten Substanzen, die u. a. gegen Achselgeruch, Fußgeruch oder Schuppenbildung eingesetzt werden.

Auch können erfindungsgemäße kosmetische und pharmazeutische, bevorzugt dermatologische Zubereitungen in zahlreichen Fällen vorteilhaft ein, zwei, drei, vier oder mehrere Konservierungsmittel enthalten. Vorzugsweise gewählt werden hierbei Konservierungsmittel wie Benzoesäure und ihre Ester und Salze, Propionsäure und ihre Ester und Salze, Salicylsäure, und ihre Ester und Salze, 2,4-Hexadiensäure (Sorbinsäure) und ihre Ester und Salze, Formaldehyd und Paraformaldehyd, 2-Hydroxybiphenylether und seine Salze, 2-Zinksulfidopyridin-N-oxid, anorganische Sulfite und Bisulfite, Natriumjodat, Chlorbutanolum, 4-Ethylquecksilber-(II)5-Amino-1,3-bis(2-Hydroxybenzoesäure, ihre Salze und Ester, Dehydratcetsäure, Ameisensäure, 1,6-Bis(4-amidino-2-bromphenoxy)-n-hexan und seine Salze, das Natriumsalz der Ethylquecksilber-(II)-thiosalicylsäure, Phenylquecksilber und seine Salze, 10-Undecylensäure und ihre Salze, 5-Amino-1,3-bis(2-ethylhexyl)-5-methylhexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxy-diphenylether, 4-Chlor-3,5-dimethylphenol, 1,1'-Methylen-bis(3-(1-hydroxymethyl-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1(4-Chlorphenoxy)1(1H-imidazol-1-yl)-3,3-dimethyl-2-butanon, 1,3-Bis-(hydroxy-methyl)-5,5-dimethyl-2,4-imidazolidindion, Benzylalkohol, Octopirox, 1,2-Dibrom-2,4-dicyanobutan, 2,2'-Methylen-bis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl(C₁₂-C₂₂)trimethyl-ammoniumbromid und -chlorid, 4,4-Dimethyl-1,3-oxazolidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-(C₈-C₁₈)-dimethyl-benzyl-ammoniumchlorid, Alkyl-(C₈-C₁₈)-dimethyl-benzylammonium-bromid, Alkyl-(C₈-C₁₈)-dimethyl-benzylammoniumsaccharinat, Benzylhemiformal, 3-Jod-2-propinyl-butylcarbamat, Natrium-hydroxymethyl-aminoacetat oder Natrium-hydroxymethyl-aminoacetat.

Erfindungsgemäße kosmetische und pharmazeutische, bevorzugt dermatologische Zubereitungen können, insbesondere wenn kristalline oder mikrokristalline Festkörper, beispielsweise anorganische Mikropigmente in die Zubereitungen eingearbeitet werden sollen, auch ein, zwei, drei, vier oder mehrere anionische, kationische, nichtionische und/oder amphotere Tenside enthalten. Tenside sind amphiphile Stoffe, die organische, unpolare Substanzen in Wasser lösen können. Bei den hydrophilen Anteilen eines Tensidmoleküls handelt es sich dabei meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nicht-ionische Tenside bilden in wässrigem Medium keine Ionen.

### A. Anionische Tenside

Vorteilhaft zu verwendende anionische Tenside sind Acylaminosäuren (und deren Salze), wie
- Acylglutamate, beispielsweise Natriumacylglutamat, Di-TEA-palmitoyl-aspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-Iauroylsarcosinat, Natriumlauroylsarcosinat und Natriumcocoylsarkosinat,
- Taurate, beispielsweise Natriumlauroyltaurat und Natriummethylcocoyltaurat,
- Acyllactylate, Lauroyllactylat, Caproyllactylat
- Alaninate

Carbonsäuren und Derivate, wie
beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
- Acyl-isothionate, z. B. Natrium-/ Ammoniumcocoyl-isethionat,
- Alkylarylsulfonate,
- Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
- Alkylethersulfat, beispielsweise Natrium-, Ammonium-, Magnesium-, MIPA, TIPA-Laurethsulfat, Natriummyrethsulfat und Natrium C12-13 Parethsulfat,
- Alkylsulfate, beispielsweise Natrium-, Ammonium- und TEA- Laurylsulfat.

### B. Kationische Tenside

Vorteilhaft zu verwendende kationische Tenside sind
- Alkylamine,
- Alkylimidazole,
- Ethoxylierte Amine und
- Quaternäre Tenside.

   RNH₂CH₂CH₂COO⁻ (bei pH=7)

   RNHCH₂CH₂COO- B⁺ (bei pH=12) B⁺ = beliebiges Kation, z.B. Na⁺
- Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benzyltrialkyl-ammoniumchloride oder -bromide, wie beispielsweise Benzyldimethylstearyl-ammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder - bromide, Alkylamidethyltrimethyl-ammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethyl-ammoniumsalze zu verwenden.

### C. Amphotere Tenside

Vorteilhaft zu verwendende amphotere Tenside sind
- Acyl-/dialkylethylendiamin, beispielsweise Natriumacylamphoacetat, Dinatriumacylamphodipropionat, Dinatriumalkylamphodiacetat, Natriumacylamphohydroxy-propylsulfonat, Dinatriumacylamphodiacetat und Natriumacylamphopropionat,
- N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
- Alkohole,
- Alkanolamide, wie Cocamide MEA/ DEA/ MIPA,
- Aminoxide, wie Cocoamidopropylaminoxid,
- Ester, die durch Veresterung von Carbonsäuren mit Ethylenoxid, Glycerin, Sorbitan oder anderen Alkoholen entstehen,
- Ether, beispielsweise ethoxylierte/propoxylierte Alkohole, ethoxylierte/ propoxylierte Ester, ethoxylierte/ propoxylierte Glycerinester, ethoxylierte/ propoxy-lierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid undCocoglycosid.
- Sucroseester, -Ether
- Polyglycerinester, Diglycerinester, Monoglycerinester
- Methylglucosester, Ester von Hydroxysäuren

Vorteilhaft ist ferner die Verwendung einer Kombination von ein, zwei, drei, vier oder mehreren anionischen und/oder amphoteren Tensiden mit einem oder mehreren nichtionischen Tensiden.

Die Gesamtmenge der oberflächenaktiven Verbindungen kann im Bereich von 1 bis 98 Gew.-% bezogen auf die erfindungsgemäßen Zubereitungen vorliegen.

Erfindungsgemäße kosmetische und pharmazeutische, bevorzugt dermatologische Zubereitungen können auch als Emulsionen vorliegen.

Die Ölphase kann vorteilhaft gewählt werden aus folgender Substanzgruppe:
- Mineralöle, Mineralwachse
- fette Öle, Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Vorteilhaft einsetzbar sind (a) Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, (b) Ester aus aromatischen Carbonsäuren und gesättigten und/oder ungesättigten, verzweigten und/oder unverzweigten Alkoholen einer Kettenlänge von 3 bis 30 C-Atomen. Bevorzugte Esteröle sind Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 3,5,5-Trimethylhexyl-3,5,5-trimethylhexanoat, 2-Ethylhexylisononanoat, 2-Ethylhexyl-3,5,5-trimethylhexanoat, 2-Ethylhexyl-2-ethylhexanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr. Auch beliebige Abmischungen solcher Öl- und Wachskomponenten sind vorteilhaft einsetzbar. In manchen Fällen ist es auch vorteilhaft, Wachse, beispielsweise Cetylpalmitat, als alleinige Lipidkomponente der Ölphase einzusetzen, vorteilhaft wird die Ölphase gewählt aus der Gruppe , die besteht aus 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid und Dicaprylylether. Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat. Auch die Kohlenwasserstoffe Paraffinöl, Squalan und Squalen lassen sich vorteilhaft verwenden. Vorteilhaft kann die Ölphase ferner einen Gehalt an cyclischen oder linearen Silikonölen aufweisen oder vollständig aus solchen Ölen bestehen, wobei es allerdings bevorzugt ist, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcyclotrisiloxan, Polydimethylsiloxan und Poly(methyl-phenylsiloxan). Besonders vorteilhaft sind ferner Mischungen aus Cyclomethicon und Isotridecylisononanoat sowie aus Cyclomethicon und 2-Ethylhexylisostearat.

Die wässrige Phase von als Emulsion vorliegenden erfindungsgemäßen kosmetischen und dermatologischen Zubereitungen, die erfindungsgemäß antiinflammatorisch wirksames 3-(4-Hydroxy-3-methoxyphenyl)-1-(4-hydroxyphenyl)-1-propanon der Formel 1 enthalten, kann vorteilhafterweise weiter umfassen: Alkohole, Diole oder Polyole niedriger C-Zahl, sowie deren Ether, vorzugsweise Ethanol, Isopropanol, Propylenglykol, Glycerin, Ethylenglykol, Ethylenglykol- monoethyl- oder -monobutylether, Propylenglykolmonomethyl, -monoethyl- oder -monobutylether, Diethylenglykolmonomethyl- oder - monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropyl-methylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Als Emulsion vorliegende erfindungsgemäße kosmetische und pharmazeutische, bevorzugt dermatologische Zubereitungen umfassen vorteilhaft einen, zwei, drei, vier oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z. B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-COO-(-CH₂-CH₂-O-)ₙ-OOH, und n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H
- der Polypropylenglycolether der allgemeinen Formel

   R-O-(-CH₂-CH(CH₃)-O-)ₙ-R'
- der propoxylierten Wollwachsalkohole,
- der veretherten Fettsäurepropoxylate R-COO-(-CH₂-CH(CH₃)-O-)ₙ-R'
- der veresterten Fettsäurepropoxylate der allgemeinen Formel

   R-COO-(-CH₂-CH(CH₃)-O-)ₙ-C(O)-R'
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)-O-)ₙ-CH₂-COOH,
- der Alkylethersulfate bzw. die diesen Sulfaten zugrundeliegenden Säuren der allgemeinen Formel R-O-(-CH₂-CH(CH₃)-O-)ₙ-SO₃-H,
- der Fettalkoholethoxylate/propoxylate der allgemeinen Formel R-O-Xₙ-Yₘ-H
- der Polypropylenglycolether der allgemeinen Formel R-O-Xₙ-Yₘ-R'
- der veretherten Fettsäurepropoxylate der allgemeinen Formel R-COO-Xₙ-Yₘ-R'
- der Fettsäureethoxylate/propoxylate der allgemeinen Formel R-COO-Xₙ-Yₘ-H.

Erfindungsgemäß besonders vorteilhaft werden die ein, zwei, drei, vier oder mehrere eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearyl-ether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)iso-cetylether (Isoceteth-17), Polyethylenglycol(18)isocetylether (Isoceteth-18), Polyethylenglycol(19)isocetylether(Isoceteth-19), Polyethylenglycol(20)isocetylether (Isoceteth-20), Polyethylenglycol(12)oleylether (Oleth-12), Polyethylenglycol(1 3)oleylether (Oleth-13), Polyethylenglycol(1 4)oleylether (Oleth-14), Polyethylenglycol(15)oleylether (Oleth-15), Polyethylenglycol(12)laurylether (Laureth-12), Polyethylenglycol(12)isolaurylether (Isolaureth12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycol-ester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether(Steareth-2), Glycerylmonolaurat, Glycerylmonocaprinat, Glycerylmonocaprylat.

Erfindungsgemäße kosmetische und pharmazeutische, bevorzugt dermatologische Zubereitungen zur topischen prophylaktischen oder kosmetischen Behandlung der Haut können regelmäßig einen hohen Anteil an pflegenden Substanzen enthalten. Gemäß einer bevorzugten Ausführungsform enthalten die Zusammensetzungen ein oder mehrere pflegende tierische und/oder pflanzliche Fette und Öle wie Olivenöl, Sonnenblumenöl, raffiniertes Sojaöl, Palmöl, Sesamöl, Rapsöl, Mandelöl, Borretschöl, Nachtkerzenöl, Kokosöl, Sheabutter, Jojobaöl, Haferöl, Spermöl, Rindertalg, Klauenöl und Schweineschmalz sowie gegebenenfalls weitere pflegende Bestandteile wie zum Beispiel Fettalkohole mit 8-30 C-Atomen. Die Fettalkohole können hierbei gesättigt oder ungesättigt bzw. linear oder verzweigt sein. Einsetzbar sind zum Beispiel Decanol, Decenol, Octanol, Octenol, Dodecanol, Dodecenol, Octadienol, Decadienol, Dodecadienol, Oleylalkohol, Ricinolalkohol, Erucaalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei die Aufzählung durch weitere strukturchemisch verwandte Alkohole nahezu beliebig erweiterbar wäre. Die Fettalkohole stammen bevorzugt von natürlichen Fettsäuren ab, wobei sie üblicherweise aus den korrespondierenden Estern der Fettsäuren durch Reduktion hergestellt werden. Einsetzbar sind weiterhin Fettalkoholfraktionen, die durch Reduktion aus natürlich vorkommenden Fetten und fetten Ölen, wie z. B. Rindertalg, Erdnußöl, Rüböl, Baumwollsamenöl, Sojaöl, Sonnenblumenöl, Palmkernöl, Leinöl, Maisöl, Rizinusöl, Rapsöl, Sesamöl, Kakaobutter und Kokosfett entstehen.

Zu pflegenden Substanzen, die sich vorzüglich mit erfindungsgemäßen Zubereitungen kombinieren lassen, zählen darüber hinaus auch
- Wachse wie z. B. Candelillawachs oder Karnaubawachs
- Ceramide, wobei man unter Ceramiden N-Acylsphingosine (Fettsäreamide des Sphingosins) oder synthetische Analoga solcher Lipide (sogenannte Pseudo-Ceramide) versteht, die das Wasserhaltevermögen des Stratum Corneums deutlich verbessern.
- Phospholipide, beispielsweise Sojalecithin, Ei-Lecithin und Kephaline
- Vaseline, Paraffin- und Silikonöle; zu letzteren zählen unter anderem Dialkyl- und Alkylarylsiloxane wie Dimethylpolysiloxan und Methylphenylpolysiloxan, sowie deren alkoxylierte und quaternierte Derivate.

Das erfindungsgemäße Verfahren zur kosmetischen Behandlung oder Vorbeugung inflammatorischer Prozesse der Haut ist bevorzugt **dadurch gekennzeichnet, dass** die erfindungsgemäße kosmetische oder pharmazeutische Zubereitung auf eine oder mehrere Hautpartien appliziert wird, die eine Inflammation und/oder die keine Inflammation aufweisen.

Die erfindungsgemäß einzusetzende Verbindung der Formel 1 kann vorzugsweise durch ein Herstellverfahren erhalten werden, welches folgende Schritte umfasst:
(a) Umsetzung (Aldol-Kondensation) von p-Hydroxyacetophenon mit Vanillin zu 3-(4-Hydroxy-3-methoxyphenyl)-1-(4-hydroxyphenyl)-2-propen-1-on,
(b) Reduktion, vorzugsweise Hydrierung, des in Schritt (a) erhaltenen Reaktionsprodukts.

Das ungesättigte Keton 3-(4-Hydroxy-3-methoxyphenyl)-1-(4-hydroxyphenyl)-2-propen-1-on ist aus der Literatur bekannt, z.B. aus DE 1 099 732, DE 1 447 016 oder auch DE 2 256 961.

Bevorzugte Ausgestaltungen und weitere Aspekte der vorliegenden Erfindung ergeben sich aus den beigefügten Patentansprüchen und den nachfolgenden Beispielen, wobei die Beispiele die Erfindung nicht beschränken sollen. Sofern nicht anders angegeben, beziehen sich alle Angaben auf das Gewicht.

### Beispiel 1: Synthese von 3-(4-Hydroxy-3-methoxyphenyl)-1-(4-hydroxyphenyl)-1-propanon, Verbindung der Formel 1

### (a) Umsetzung von p-Hydroxyacetophenon mit Vanillin:

Es werden 20 g Kaliumhydroxid in 100 g Diethylenglykoldiethylether vorgelegt, auf 120°C unter Rühren erhitzt und mit einer Mischung von 14 g p-Hydroxyacetophenon und 15 g Vanillin innerhalb von 1 h versetzt. Nach beendeter Dosierung rührt man noch 20 min nach, hydrolysiert und stellt auf pH 6-7 ein. Nach Phasentrennung entfernt man das Lösungsmittel und erhält 25 g 3-(4-Hydroxy-3-methoxyphenyl)-1-(4-hydroxyphenyl)-2-propen-1-on, Ausbeute: 93% der Theorie.

### (b) Hydrierung des in Schritt (a) gewonnenen Reaktionsprodukts:

10 g 3-(4-Hydroxy-3-methoxyphenyl)-1-(4-hydroxyphenyl)-2-propen-1-on werden in 100 g Tetrahydrofuran gelöst, mit 0,2 g Pd auf Aktivkohle (Pd-Gehalt: 5 Gew.-%, Wassergehalt ca. 50 Gew.-%, jeweils bezogen auf die Gesamtmasse des Katalysators) versetzt und bei Normaldruck und Raumtemperatur (ca. 20°C) hydriert. Man erhält nach Entfernung des Katalysators und des Lösungsmittels 9 g an 3-(4-Hydroxy-3-methoxyphenyl)-1-(4-hydroxyphenyl)-propan-1-on. Ausbeute: 89% der Theorie.

Spektroskopische Daten von 3-(4-Hydroxy-3-methoxyphenyl)-1-(4-hydroxyphenyl)-propan-1-on (Formel 1):
¹³C-NMR (CDCl₃; 75,5 MHz): δ (ppm) = 197,42(s), 161,85(s), 147,24(s), 144,44(s), 132,03(s), 130,37(d), 130,37(d), 128,18(s), 120,27(d), 115,11(d), 115,07(d), 115,07(d), 112,53(d), 55,42(q), 39,30(t), 29,42(t);
MS: m/z (%) = M⁺-Ion 272(82), 151(24), 137(77), 121(100), 93(19), 65(22).

### Beispiele 2 - 11: Kosmetische Formulierungen enthaltend 3-(4-Hydroxy-3-methoxyphenyl)-1-(4-hydroxyphenyl)-1-propanon der Formel 1

Formulierungen enthaltend erfindungsgemäßes, antiinflammatorisch wirksames 3-(4-Hydroxy-3-methoxyphenyl)-1-(4-hydroxyphenyl)-1-propanon.

In der nachfolgenden Tabelle 2 bedeuten
**1 = Hautaufhellende Tagescreme O/W**
**2 = Hautberuhigende Lotion mit Pflanzenextrakten O/W**
**3 = After Sun Balm**
**4 = Körperspray**
**5 = Sonnenschutzlotion (O/W), Breitbandschutz**
**6 = W/O Nachtcreme**
**7 = Shampoo**
**8 = Selbstbräunungscreme**
**9 = Barrierereparaturcreme O/W**
**10 = Antitranspirant/Deodorant Roll-on**

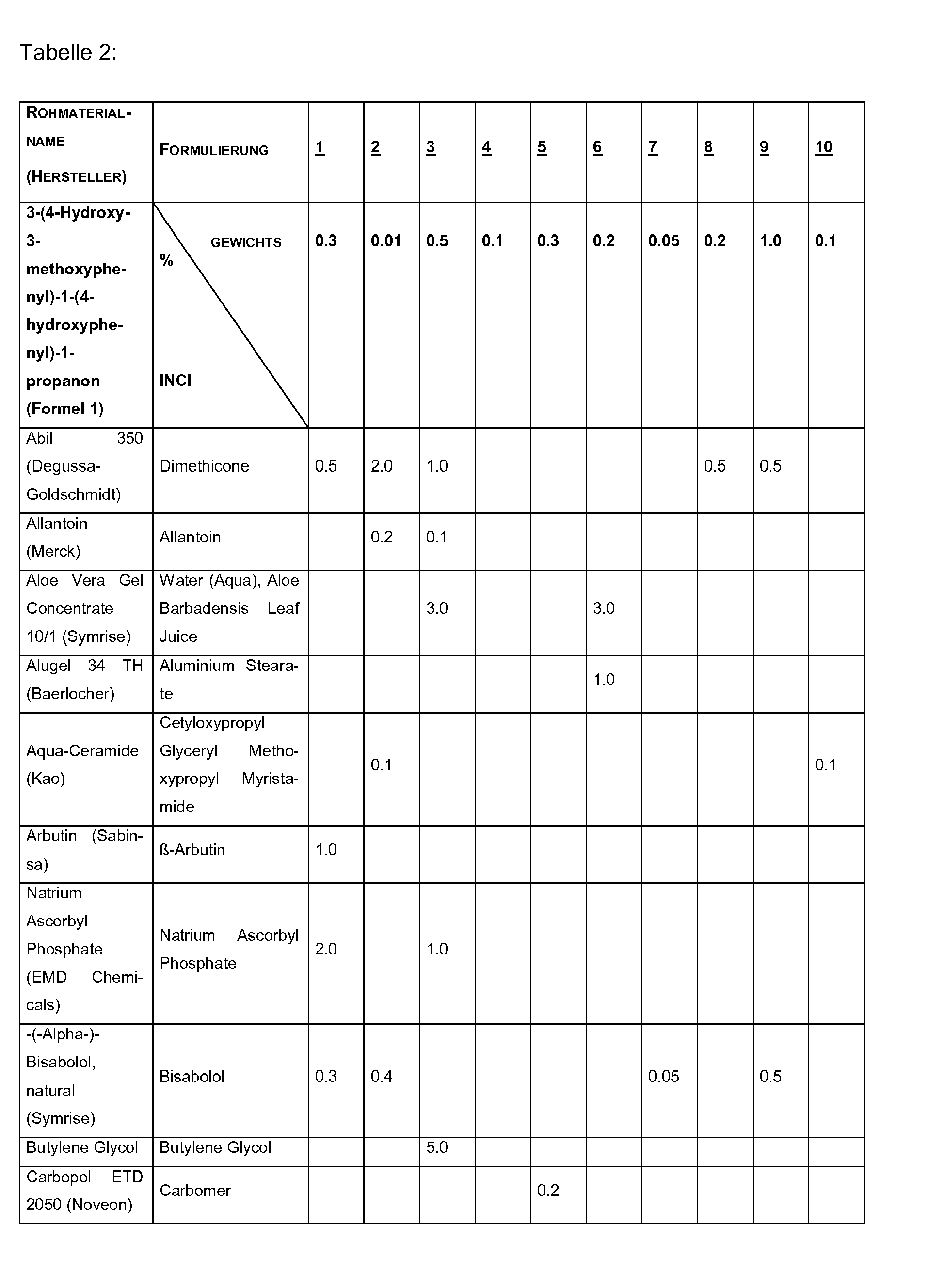

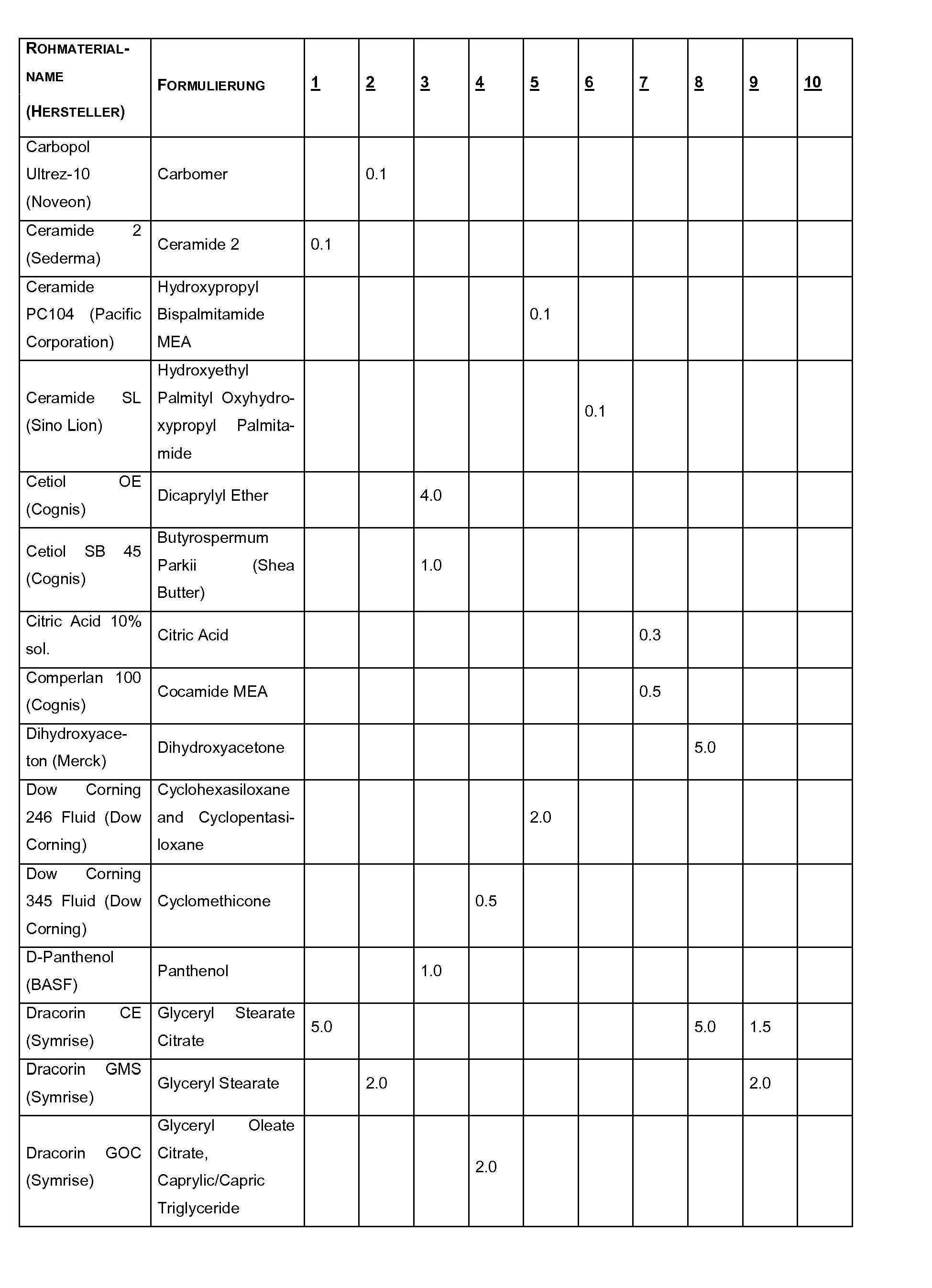

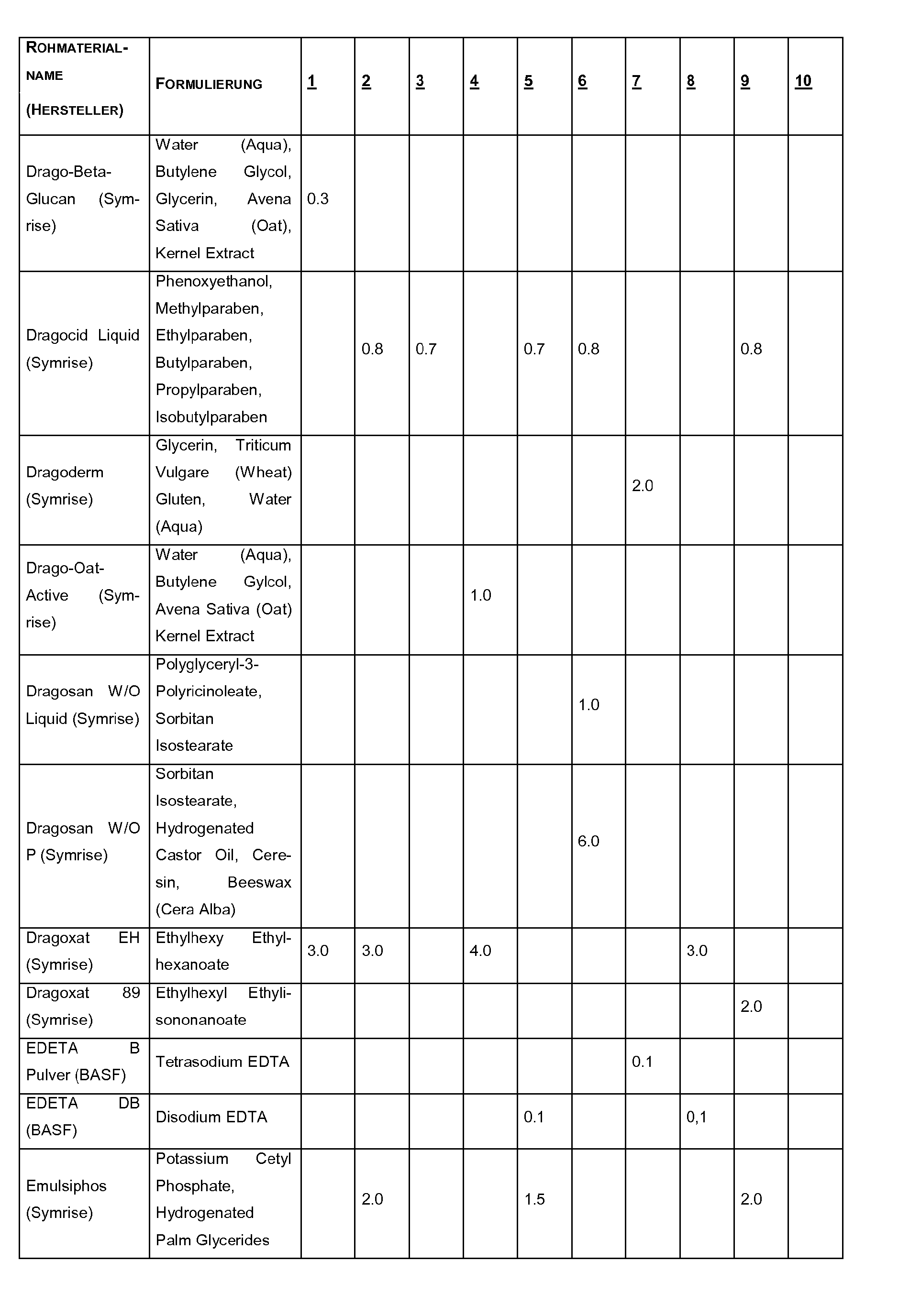

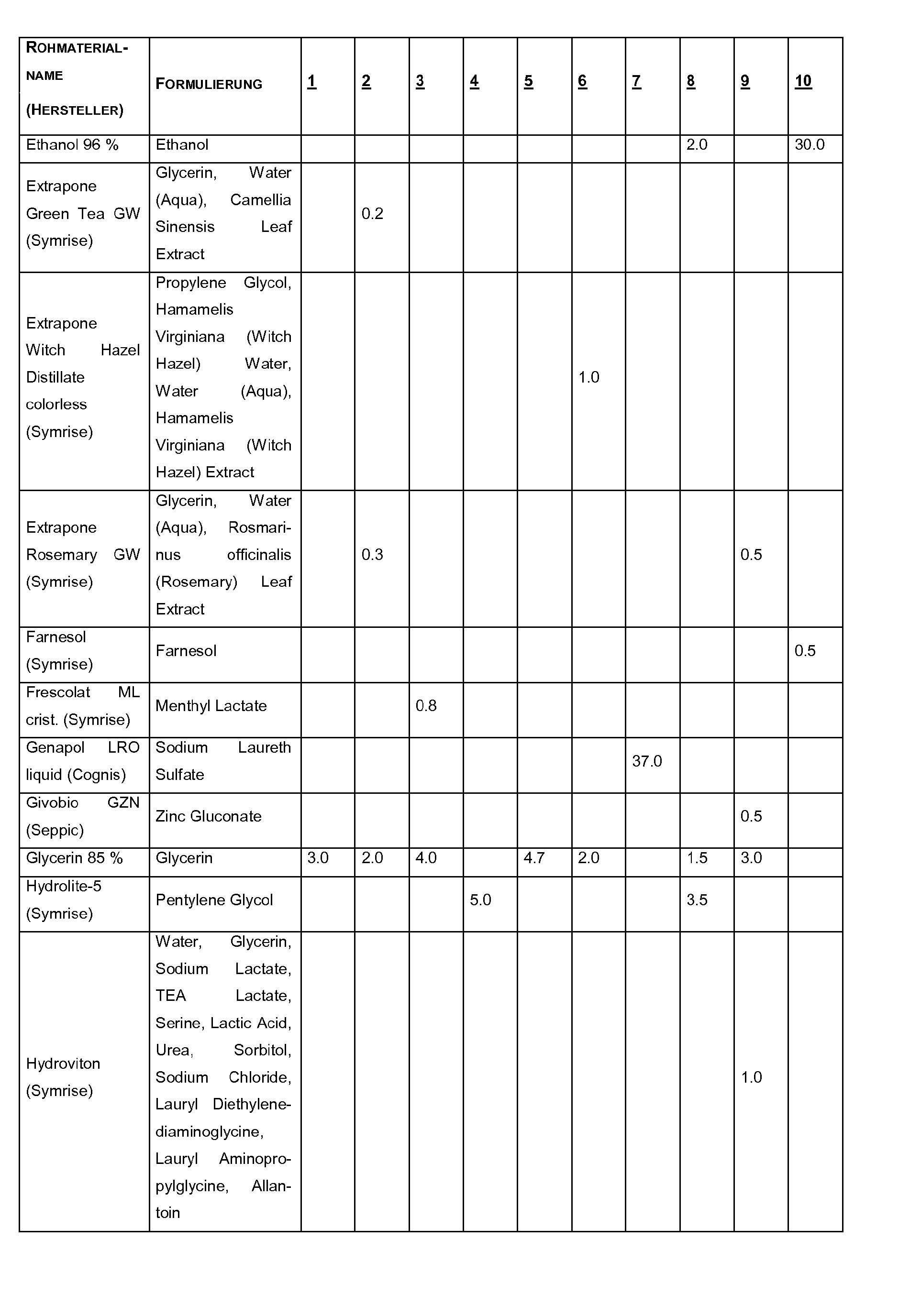

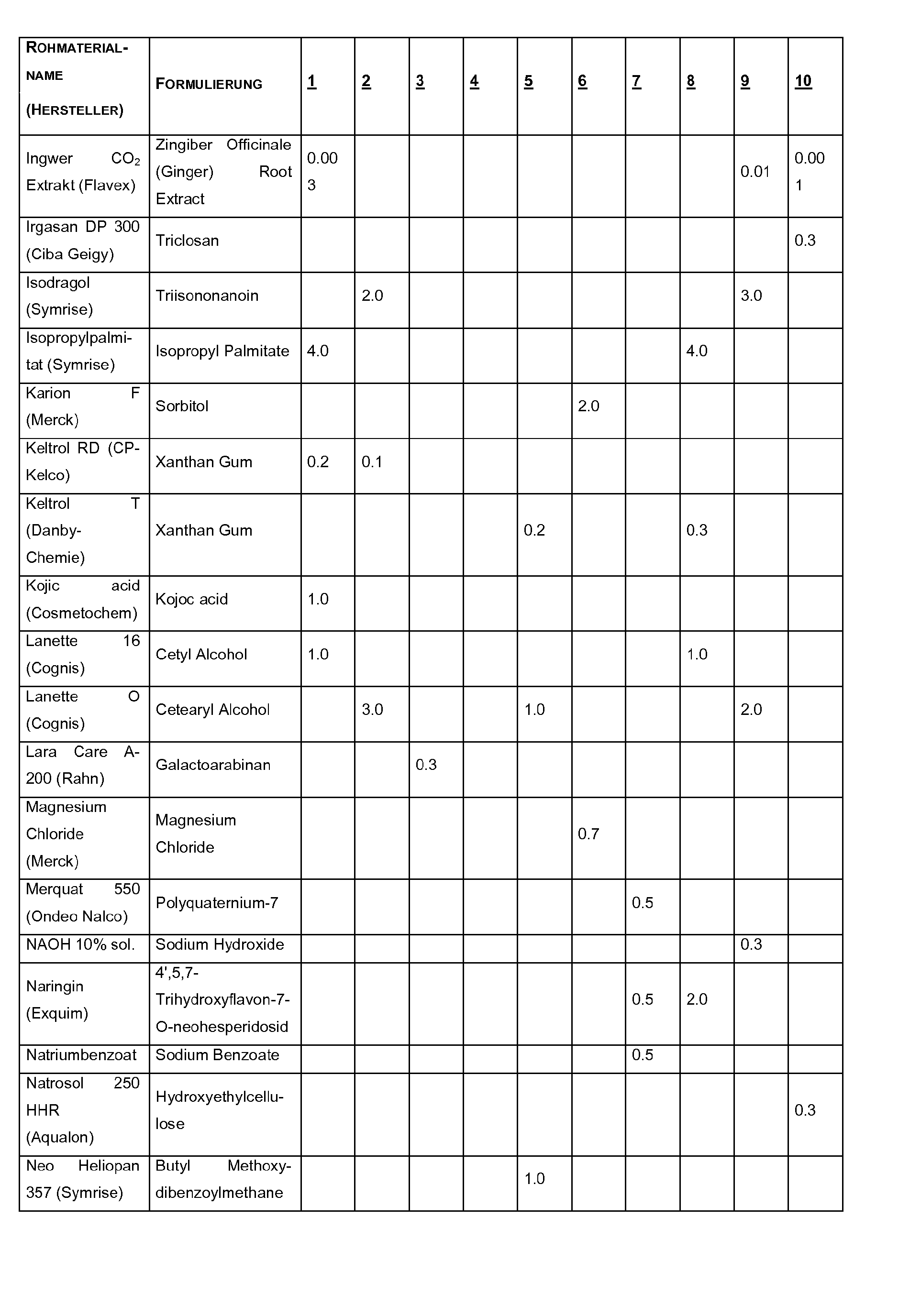

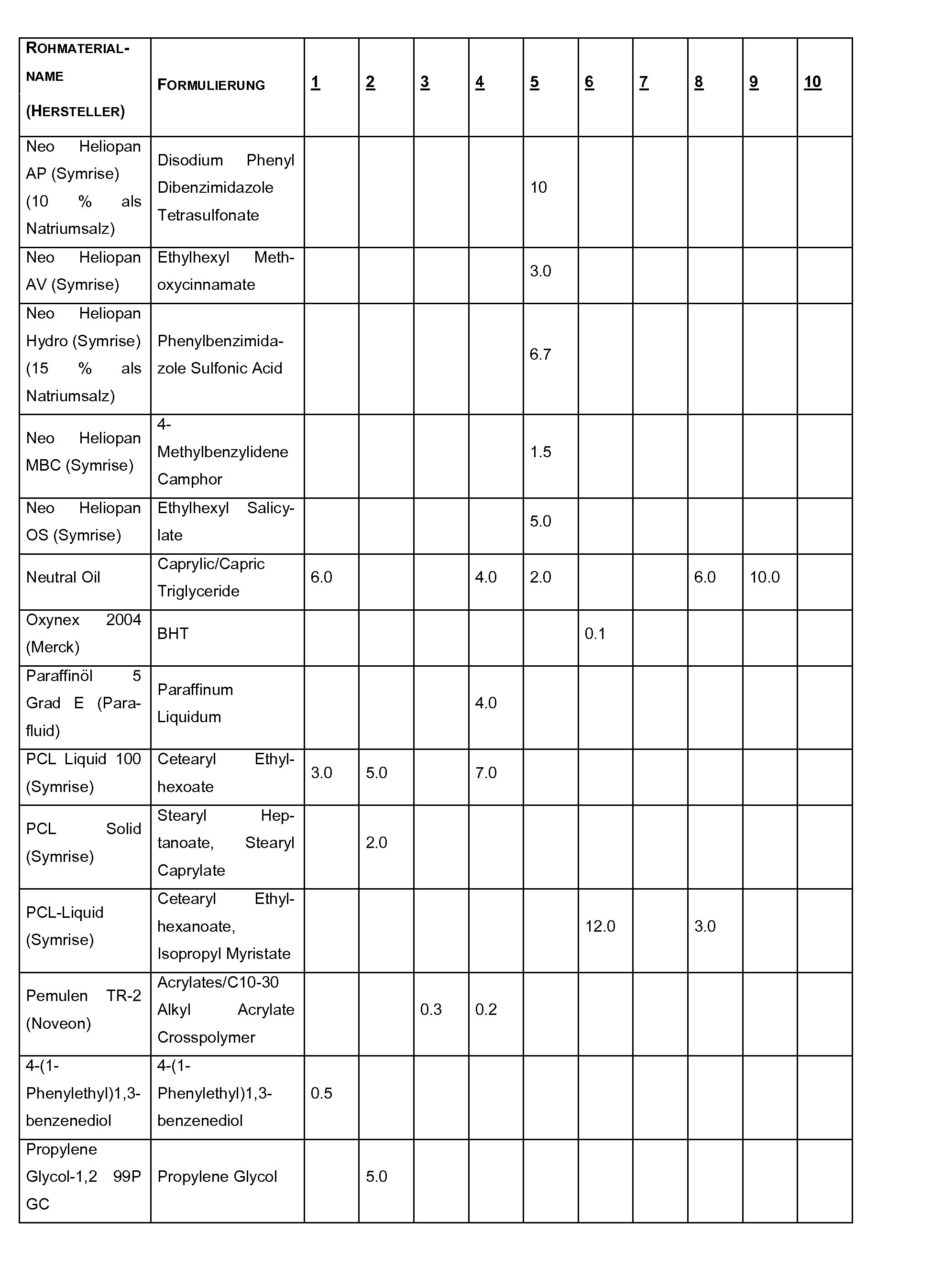

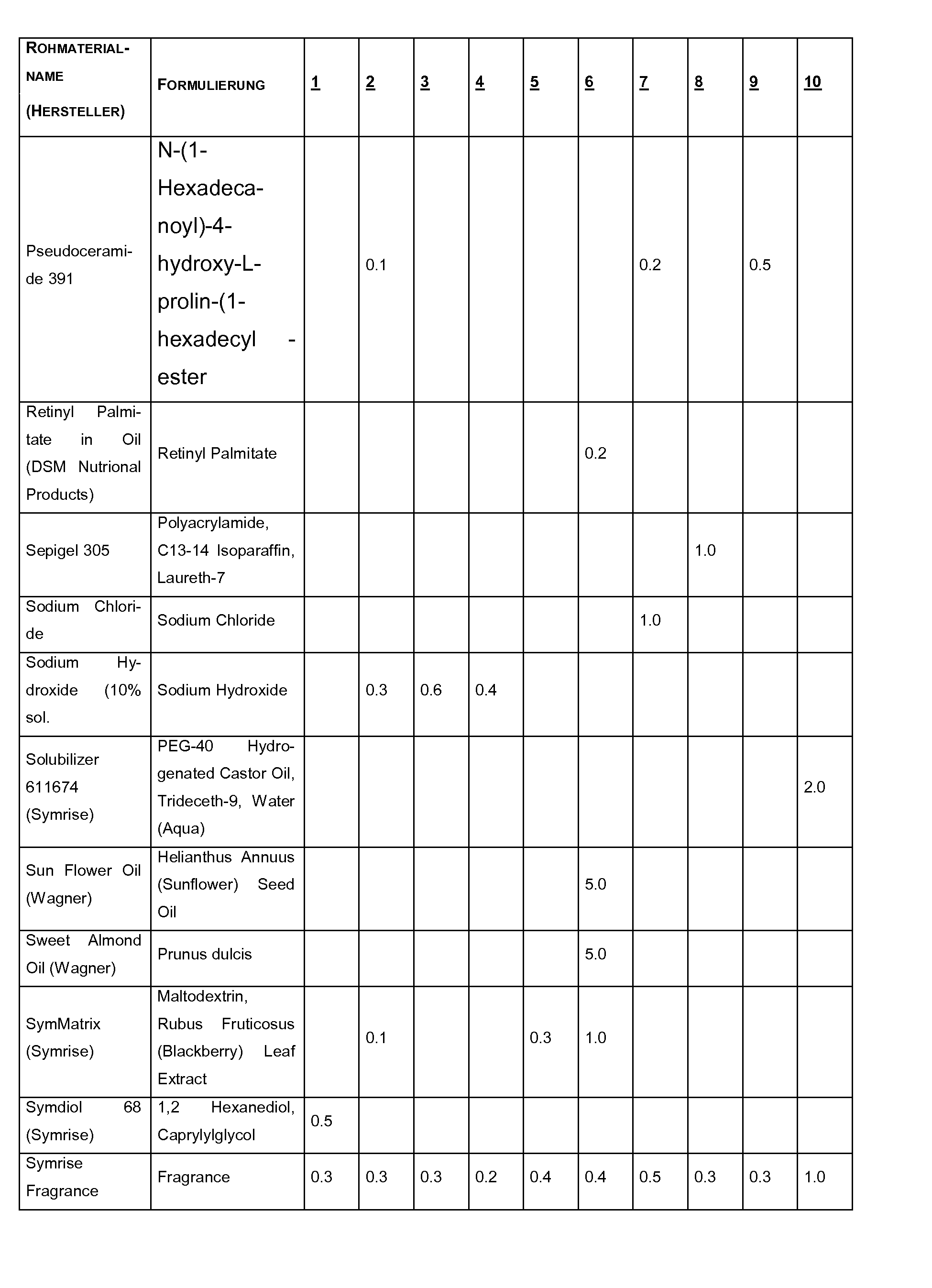

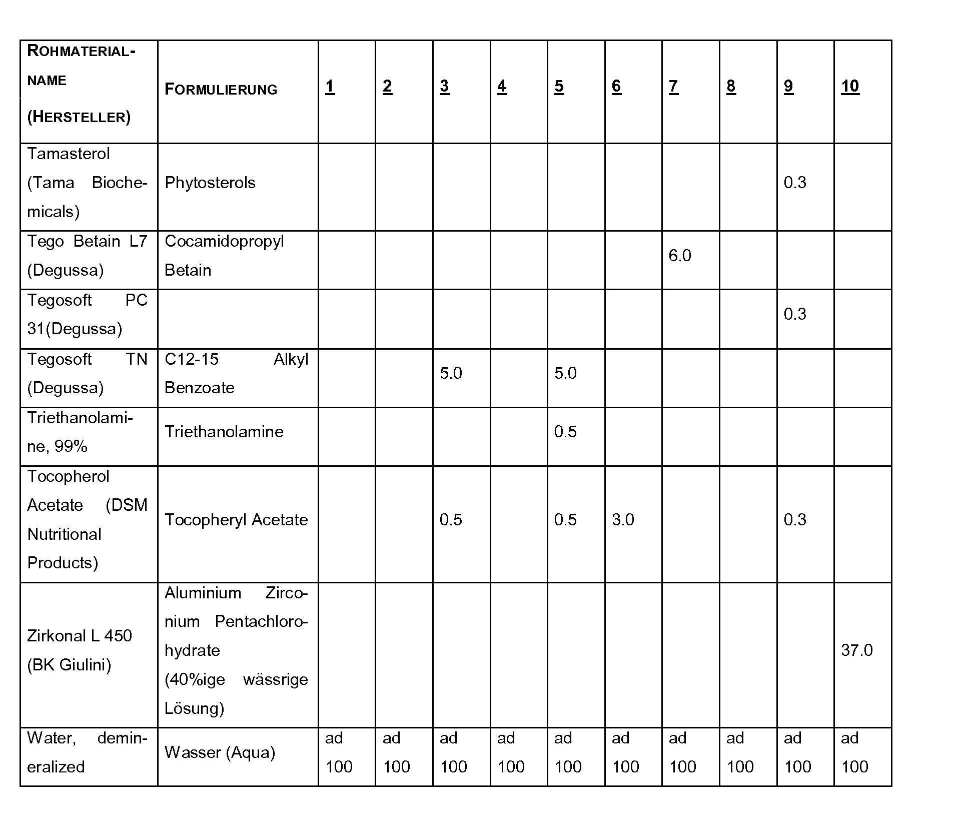

## Patentansprüche

1. Kosmetische oder pharmazeutische Zubereitung umfassend oder bestehend aus den folgenden Komponenten:
a) eine antiinflammatorisch wirksame Menge der Verbindung der Formel 1 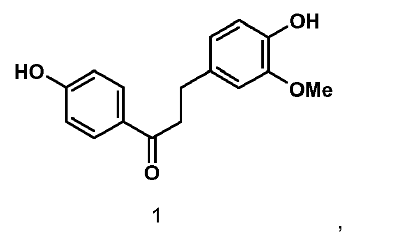
b) gegebenenfalls ein, zwei, drei, vier oder mehrere weitere antiinflammatorisch wirksame Verbindungen,
sowie
c) eine oder mehrere zu Komponenten (a) und (b) kompatible kosmetische und/oder pharmazeutische Grund-, Hilfs- oder Zusatzstoffe.

2. Zubereitung gemäß Anspruch 1, **dadurch gekennzeichnet, dass** die Zubereitung zusätzlich
d) eine wirksame Menge eines, zwei, drei, vier oder mehrerer UV-Filter umfasst, so dass der Lichtschutzfaktor der kosmetischen oder pharmazeutischen Zubereitung >2 beträgt.

3. Zubereitung gemäß Anspruch 1 oder 2, **dadurch gekennzeichnet, dass** die Zubereitung zusätzlich
e) ein, zwei, drei, vier oder mehrere Verbindungen in einer Menge bezogen auf die fertige Zubereitung umfasst, die in einer Zubereitung ohne die Verbindung der Formel 1 eine inflammatorische Wirkung auf die menschliche oder tierische Haut vermittelt,
wobei die Menge der Verbindung der Formel 1 bezogen auf die fertige Zubereitung ausreicht, die inflammatorische Wirkung der Verbindungen aus e) zu verringern, aufzuheben oder zu unterdrücken.

4. Zubereitung gemäß einem der Ansprüche 1 bis 3, **dadurch gekennzeichnet, dass** die Zubereitung ausgewählt wird aus der Gruppe bestehend aus flüssige Formulierungen, bevorzugt Reinigungslösungen oder Tränkflüssigkeiten; halbfeste Formulierungen, bevorzugt Salben und Cremes.

5. Verbindung der Formel 1 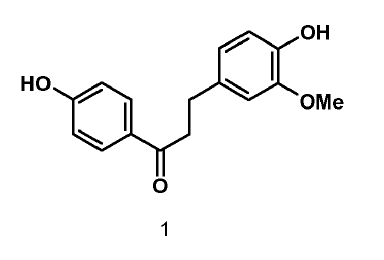 zur Verwendung als Arzneimittel.

6. Verbindung der Formel 1 gemäß Anspruch 5, **dadurch gekennzeichnet, dass** das Arzneimittel topisch appliziert wird.

7. Verbindung der Formel 1 gemäß Anspruch 6, **dadurch gekennzeichnet, dass** Arzneimittel zur Behandlung von Inflammationen der Haut verwendet wird.

8. Verwendung einer Verbindung der Formel 1 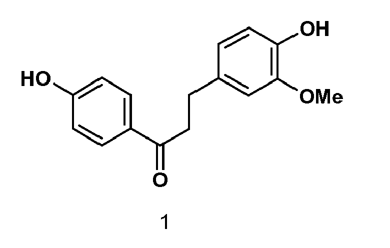 in einer antiinflammatorisch wirksamen Menge zur Behandlung oder Vorbeugung von Inflammationen.

9. Verwendung einer Verbindung der Formel 1 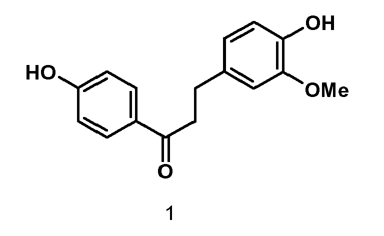 in einer antiinflammatorisch wirksamen Menge in kosmetischen Zubereitungen.

10. Verwendung einer Verbindung der Formel 1 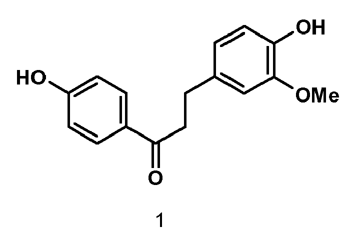 in einer antiinflammatorisch wirksamen Menge zur Herstellung eines Arzneimittels zur Behandlung oder Vorbeugung von Inflammationen.

11. Verwendung einer antiinflammatorisch wirksamen Menge einer Verbindung der Formel 1 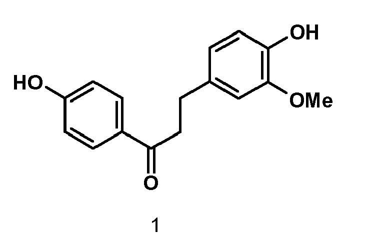 oder einer kosmetischen oder pharmazeutischen Zubereitung gemäß einem der Ansprüche 1 bis 4 zum Verringern, Aufheben oder Unterdrücken einer inflammatorischen Wirkung einer, zwei, drei, vier oder mehreren Verbindungen auf die menschliche oder tierische Haut.

12. Verfahren zur kosmetischen Behandlung oder Vorbeugung inflammatorischer Prozesse der Haut umfassend oder bestehend aus der topischen Applikation einer antiinflammatorisch wirksamen Menge der Zubereitung gemäß einem der Ansprüche 1 bis 4 auf die Haut eines menschlichen oder tierischen Körpers.

13. Verfahren gemäß Anspruch 12, **dadurch gekennzeichnet, dass** die Zubereitung auf eine oder mehrere Hautpartien appliziert wird, die eine Inflammation und/oder die keine Inflammation aufweisen.

14. Verfahren zum Behandeln oder Vorbeugen einer inflammatorischen Wirkung einer, zwei, drei, vier oder mehreren Verbindungen auf die menschliche oder tierische Haut umfassend oder bestehend aus folgenden Schritten:
a) Bereitstellen einer, zwei, drei, vier oder mehreren Verbindungen, die eine inflammatorische Wirkung auf die Haut ausüben,
b) Bereitstellen einer Verbindung der Formel 1 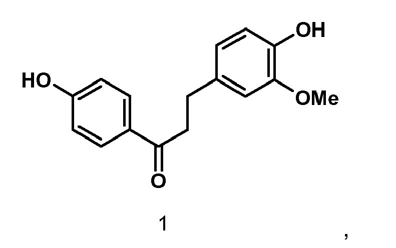
c) gegebenenfalls Bereitstellen eines, zwei, drei, vier oder mehrerer Grund-, Hilfs- und/oder Zusatzstoffe für kosmetische oder pharmazeutische Zubereitungen und
d) Vermischen der Verbindungen aus Schritt a) mit der Verbindung der Formel 1 aus Schritt b) und gegebenenfalls der Grund-, Hilfs- und/oder Zusatzstoffe aus Schritt c) zu einer Zubereitung,
wobei ein oder mehrere Verbindungen aus Schritt a) in einer Menge bezogen auf die fertige Zubereitung umfasst sind, die in einer Zubereitung ohne die Verbindung der Formel 1 eine inflammatorische Wirkung auf die menschliche oder tierische Haut vermitteln und
wobei die Verbindung der Formel 1 in einer Menge umfasst ist, die ausreicht die inflammatorische Wirkung der Verbindungen aus Schritt a) auf die Haut zu verringern, aufzuheben oder zu unterdrücken.

15. Duftstoffkomposition, umfassend oder bestehend aus
a) einer sensorisch wirksamen Menge eines Duftstoffes oder Duftstoffgemisches,
b) einer antiinflammatorisch wirksamen Menge einer Verbindung der Formel 1 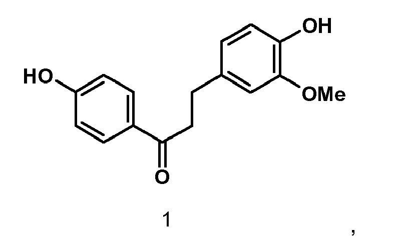
c) gegebenenfalls ein, zwei, drei, vier oder mehreren weiteren antiinflammatorisch wirksamen Verbindungen und
d) gegebenenfalls ein oder mehreren Grund-, Hilfs- und/oder Zusatzstoffen.
